# EUROPEAN PATENT APPLICATION

(11) **EP 4 372 088 A1**
(43) Date of publication of application: **22.05.2024**
(21) Application number: 22842182.2
(22) Date of filing: 14.07.2022
(51) Int. Cl.: C12N 15/62, A61K 35/17, A61K 38/19, A61K 38/20, A61K 39/395, A61K 47/68, A61K 48/00, A61P 35/00, A61P 37/04, C07K 14/54, C07K 16/28, C07K 19/00, C12N 5/0783, C12N 5/10, C12N 15/11, C12N 15/13, C12N 15/24

(54) **ANTI-EGFRVIII ANTIBODY, POLYPEPTIDE, CELL CAPABLE OF EXPRESSING SAID POLYPEPTIDE, PHARMACEUTICAL COMPOSITION CONTAINING SAID CELL, METHOD FOR PRODUCING SAID CELL, AND POLYNUCLEOTIDE OR VECTOR COMPRISING NUCLEOTIDE SEQUENCE ENCODING SAID POLYPEPTIDE**

(30) Priority: 16.07.2021 JP 2021118056
(71) Applicant: Noile-Immune Biotech Inc., Tokyo 105-0012 (JP)
(72) Inventor: TAMADA, Koji, Ube-shi, Yamaguchi 755-8505 (JP); SAKODA, Yukimi, Ube-shi, Yamaguchi 755-8505 (JP); ADACHI, Keishi, Ube-shi, Yamaguchi 755-8505 (JP)
(74) Representative: Dennemeyer & Associates S.A.
(86) International application number: PCT/JP2022/027735
(87) International publication number: WO 2023/286840

(57) **Abstract**

An anti-EGFRviii antibody, a polypeptide, a cell expressing the polypeptide, a pharmaceutical composition containing the cell, a method for producing the cell, and a polynucleotide or a vector including a nucleotide sequence encoding the polypeptide.

## Description

### Technical Field

The present disclosure relates to an anti-EGFRviii antibody, a polypeptide, a cell expressing the polypeptide, a pharmaceutical composition containing the cell, a method for producing the cell, and a polynucleotide or a vector including a nucleotide sequence encoding the polypeptide.

### Background Art

A chimeric antigen receptor (hereinafter, also referred to as "CAR") is an artificial chimeric protein in which a target antigen-binding region that recognizes a cell surface antigen of a cancer cell and a signal transduction region that induces activation of T cells are fused.

For example, a large amount of CAR-expressing T cells (hereinafter, also referred to as "CAR-T cells") can be produced by introducing a gene encoding a CAR into peripheral blood T cells (peripheral blood T lymphocytes). Such CAR-T cells have tumor reactivity and can bring injury of cancer cells without depending on an interaction with a major histocompatibility complex (MHC).

In cancer immunotherapy by administration of CAR-T cells, more specifically, a therapy in which T cells are collected from a patient, a gene encoding a CAR is introduced into the T cells and amplified, and the resulting T cells are transferred again to the patient, clinical trials are currently in progress worldwide, and results indicating effectiveness in, for example, a hematopoietic organ malignant tumor such as leukemia or lymphoma have been obtained.

In the cancer immunotherapy using CAR-T cells, it is required to search for a target antigen specifically expressed in a target tumor. Furthermore, an antibody that specifically binds to the target antigen and can be used as a target antigen-binding region of a CAR is required. The antibody used as the target antigen-binding region needs to have a sufficiently high expression ratio when being expressed as a part of the CAR, and cells expressing the CAR need to exhibit a high antitumor effect. An antibody having high affinity for an antigen is not necessarily useful for the CAR, and it is not easy to search for an antibody that can be used as the target antigen-binding region.

Meanwhile, CAR-T cell therapy has been considered to be difficult to obtain an effect on a solid tumor. It is considered that this is because, for example, a survival ratio of CAR-T cells in a living body is low, accumulation of the CAR-T cells in a tumor site is low, and activity of the CAR-T cells is inhibited by an immunosuppressive factor or the like secreted by tumor cells.

As a method for solving such a problem, a method for introducing a nucleic acid encoding a T cell immune function promoting factor into a T cell together with a nucleic acid encoding a CAR has been reported. For example, JP 6761113 B2 discloses a CAR using a solid tumor antigen as a target antigen, and a CAR-T cell effective against a solid tumor.

### Summary of Invention

### Technical Problem

JP 6761113 B2 discloses a CAR-T cell that exhibits effectiveness against ganglioside GM2 as a target antigen. However, there is a demand for development of a CAR that targets an antigen other than ganglioside GM2 in a solid tumor antigen, an additional CAR-T cell effective against a solid tumor, and a peptide that can be used for the CAR-T cell.

A problem to be solved by an embodiment according to the present disclosure is to provide a novel antibody that specifically binds to EGFRviii and can also be used as a target antigen-binding region of a chimeric antigen receptor.

A problem to be solved by another embodiment according to the present disclosure is to provide a polypeptide containing the above novel antibody, a cell expressing the polypeptide, a polynucleotide or a vector including a nucleotide sequence encoding the polypeptide, and a pharmaceutical composition containing the cell.

### Solution to Problem

An embodiment of the present disclosure includes the following aspects.
<1> An anti-EGFRviii antibody which is an antibody or an antigen-binding fragment thereof selected from the group consisting of the following (a-1) to (a-3):
   (a-1) an antibody or an antigen-binding fragment thereof including:
      a heavy chain variable region including heavy chain variable regions CDR1, CDR2, and CDR3 each consisting of the amino acid sequence set forth in SEQ ID NO: 1 or 4; and
      a light chain variable region including light chain variable regions CDR1, CDR2, and CDR3 each consisting of the amino acid sequence set forth in SEQ ID NO: 2, 3, 5, or 6, and
      having binding ability to EGFRviii;
   (a-2) an antibody or an antigen-binding fragment thereof including:
      a heavy chain variable region consisting of an amino acid sequence having one or a plurality of amino acids mutated in the amino acid sequence set forth in SEQ ID NO: 1 or 4; and
      a light chain variable region consisting of an amino acid sequence having one or a plurality of amino acids mutated in the amino acid sequence set forth in SEQ ID NO: 2, 3, 5, or 6, and
      having binding ability to EGFRviii; and
   (a-3) an antibody or an antigen-binding fragment thereof including:
      a heavy chain variable region consisting of an amino acid sequence having 90% or more of sequence identity to the amino acid sequence set forth in SEQ ID NO: 1 or 4; and
      a light chain variable region consisting of an amino acid sequence having 90% or more of sequence identity to the amino acid sequence set forth in SEQ ID NO: 2, 3, 5, or 6;
         and
      having binding ability to EGFRviii.
<2> The anti-EGFRviii antibody according to <1>, in which the heavy chain variable region has T at position 21, F at position 24, F at position 54, K at position 59, S at position 67, K at position 73, T or K at position 77, and K or T at position 83 in the amino acid sequence set forth in SEQ ID NO: 1 or 4.
<3> The anti-EGFRviii antibody according to <1> or <2>, in which the light chain variable region has M at position 4, E or G at position 16, Q at position 27, K at position 35, S at position 55, N at position 58, R at position 74, K at position 79, S at position 81, D at position 87, and V at position 99 in the amino acid sequence set forth in SEQ ID NO: 2, 3, 5, or 6.
<4> The anti-EGFRviii antibody according to any one of <1> to <3>, in which
   the heavy chain variable region includes the amino acid sequence set forth in SEQ ID NO: 1 and the light chain variable region includes the amino acid sequence set forth in SEQ ID NO: 2,
   the heavy chain variable region includes the amino acid sequence set forth in SEQ ID NO: 1 and the light chain variable region includes the amino acid sequence set forth in SEQ ID NO: 3,
      or
   the heavy chain variable region includes the amino acid sequence set forth in SEQ ID NO: 4 and the light chain variable region includes the amino acid sequence set forth in SEQ ID NO: 5 or 6.
<5> The anti-EGFRviii antibody according to any one of <1> to <4>, in which the antibody is a single chain variable fragment.
<6> The anti-EGFRviii antibody according to <5>, in which the single chain variable fragment includes the light chain variable region, a peptide linker linking the heavy chain variable region and the light chain variable region to each other, and the heavy chain variable region sequentially from an N-terminus.
<7> The anti-EGFRviii antibody according to <5>, in which the single chain variable fragment is a polypeptide selected from the group consisting of the following (I) to (III) :
   (I) a polypeptide including an amino acid sequence selected from the group consisting of SEQ ID NOs: 12 to 18 and 38;
   (II) a polypeptide including an amino acid sequence having 70% or more of sequence identity to the amino acid sequence selected from the group consisting of SEQ ID NOs: 12 to 18 and 38, and having binding ability to EGFRviii; and
   (III) a polypeptide including an amino acid sequence having one or a plurality of amino acids mutated in the amino acid sequence selected from the group consisting of SEQ ID NOs: 12 to 18 and 38, and having binding ability to EGFRviii.
<8> A polypeptide including a target antigen-binding region, a transmembrane region, and a signal transduction region, in which
   the target antigen-binding region includes the anti-EGFRviii antibody according to any one of <1> to <7>.
<9> The polypeptide according to <8>, which is a chimeric antigen receptor.
<10> A cell expressing the polypeptide according to <8> or <9>.
<11> The cell according to <10>, further expressing at least one of IL-7 and CCL19, preferably further expressing at least one of exogenous IL-7 and exogenous CCL19.
<12> The cell according to <10> or <11>, in which the cell is an immune cell.
<13> The cell according to <12>, in which the immune cell is a T cell.
<14> A polynucleotide including a nucleotide sequence encoding the polypeptide according to <8> or <9>.
<15> The polynucleotide according to <14>, further including at least one of a nucleotide sequence encoding IL-7 and a nucleotide sequence encoding CCL19, preferably as an exogenous nucleic acid.
<16> A vector containing the polynucleotide according to <14> or <15>.
<17> The vector according to <16>, further including at least one of a nucleotide sequence encoding IL-7 and a nucleotide sequence encoding CCL19.
<18> A method for producing a cell expressing a polypeptide, the method including introducing the polynucleotide according to <14> or <15> into a cell.
<19> A method for producing a cell expressing a polypeptide, the method including introducing the vector according to <16> or <17> into a cell.
<20> A pharmaceutical composition containing the cell according to any one of <10> to <13>.
<21> The pharmaceutical composition according to <20>, which is a pharmaceutical composition for treating or preventing a tumor.

### Advantageous Effects of Invention

An embodiment of the present disclosure can provide a novel antibody that specifically binds to EGFRviii and can also be used as a target antigen-binding region of a chimeric antigen receptor.

Another embodiment of the present disclosure can provide a polypeptide containing the above novel antibody, a cell expressing the polypeptide, a polynucleotide or a vector including a nucleotide sequence encoding the polypeptide, and a pharmaceutical composition containing the cell.

### Brief Description of Drawings

Fig. 1A is a diagram illustrating an example of a result of analyzing an amino acid sequence of an antibody that binds to EGFRviii.
Fig. 1B is a diagram illustrating an example of a result of analyzing an amino acid sequence of an antibody that binds to EGFRviii.
Fig. 2A is a diagram illustrating an example of a result of measuring an expression level of an anti-EGFRviiiCAR by flow cytometry.
Fig. 2B is a diagram illustrating an example of a result of measuring an expression level of an anti-EGFRviiiCAR by flow cytometry.
Fig. 2C is a diagram illustrating an example of a result of measuring an expression level of an anti-EGFRviiiCAR by flow cytometry.
Fig. 2D is a diagram illustrating an example of a result of measuring an expression level of an anti-EGFRviiiCAR by flow cytometry.
Fig. 2E is a diagram illustrating an example of a result of measuring an expression level of an anti-EGFRviiiCAR by flow cytometry.
Fig. 2F is a diagram illustrating an example of a result of measuring an expression level of an anti-EGFRviiiCAR by flow cytometry.
Fig. 2G is a diagram illustrating an example of a result of measuring an expression level of an anti-EGFRviiiCAR by flow cytometry.
Fig. 2H is a diagram illustrating an example of a result of measuring an expression level of an anti-EGFRviiiCAR by flow cytometry.
Fig. 2I is a diagram illustrating an example of a result of measuring an expression level of an anti-EGFRviiiCAR by flow cytometry.
Fig. 3A is a diagram illustrating an example of a result of measuring an expression level of an anti-EGFRviiiCAR by flow cytometry.
Fig. 3B is a diagram illustrating an example of a result of measuring an expression level of an anti-EGFRviiiCAR by flow cytometry.
Fig. 3C is a diagram illustrating an example of a result of measuring an expression level of an anti-EGFRviiiCAR by flow cytometry.
Fig. 3D is a diagram illustrating an example of a result of measuring an expression level of an anti-EGFRviiiCAR by flow cytometry.
Fig. 4A is a diagram illustrating an example of a result of measuring cytotoxic activity of PRIME EGFRviii CAR-T cells.
Fig. 4B is a diagram illustrating an example of a result of measuring cytotoxic activity of PRIME EGFRviii CAR-T cells.
Fig. 5A is a diagram illustrating an example of a result of measuring an expression level of an anti-EGFRviiiCAR by flow cytometry.
Fig. 5B is a diagram illustrating an example of a result of measuring an expression level of an anti-EGFRviiiCAR by flow cytometry.
Fig. 5C is a diagram illustrating an example of a result of measuring an expression level of an anti-EGFRviiiCAR by flow cytometry.
Fig. 5D is a diagram illustrating an example of a result of measuring an expression level of an anti-EGFRviiiCAR by flow cytometry.
Fig. 5E is a diagram illustrating an example of a result of measuring an expression level of an anti-EGFRviiiCAR by flow cytometry.
Fig. 6A is a diagram illustrating an example of a result of measuring cytotoxic activity of PRIME EGFRviii CAR-T cells.
Fig. 6B is a diagram illustrating an example of a result of measuring cytotoxic activity of PRIME EGFRviii CAR-T cells.
Fig. 7 is a diagram illustrating an example of a result of measuring an IFNγ concentration of a culture supernatant when a ratio (E : T ratio) between PRIME EGFRviii CAR-T cells and EGFRviii-expressing cells (glioblastoma cells U87MGviii 4.12 cells) or EGFRviii-non-expressing cells (U87MG cells) is 1 : 3.
Fig. 8A is a diagram illustrating an example of a result of measuring an expression level of an anti-EGFRviiiCAR by flow cytometry in a case where a sample obtained from a healthy donor is used.
Fig. 8B is a diagram illustrating an example of a result of measuring an expression level of an anti-EGFRviiiCAR by flow cytometry in a case where a sample obtained from a healthy donor is used.
Fig. 8C is a diagram illustrating an example of a result of measuring an expression level of an anti-EGFRviiiCAR by flow cytometry in a case where a sample obtained from a healthy donor is used.
Fig. 8D is a diagram illustrating an example of a result of measuring an expression level of an anti-EGFRviiiCAR by flow cytometry in a case where a sample obtained from a healthy donor is used.
Fig. 9A is a diagram illustrating an example of a result of measuring cytotoxic activity of PRIME EGFRviii CAR-T cells and activated T cells targeting EGFRviii non-expressing tumor cells by ATP assay.
Fig. 9B is a diagram illustrating an example of a result of measuring cytotoxic activity of PRIME EGFRviii CAR-T cells and activated T cells targeting EGFRviii expressing tumor cells by ATP assay.
Fig. 9C is a diagram illustrating an example of a result of measuring cytotoxic activity of PRIME EGFRviii CAR-T cells and activated T cells targeting EGFRviii non-expressing tumor cells by ATP assay.
Fig. 9D is a diagram illustrating an example of a result of measuring cytotoxic activity of PRIME EGFRviii CAR-T cells and activated T cells targeting EGFRviii expressing tumor cells by ATP assay.
Fig. 10 is a diagram illustrating an example of a result of staining tumor cells with a PE-labeled anti-human EGFRviii antibody and performing flow cytometry analysis.
Fig. 11 is a diagram illustrating an example of a photograph of a back of a tumor model mouse to which tumor cells have been transplanted.
Fig. 12A is a graph illustrating an example of a result of a change in tumor volume of a tumor model mouse.
Fig. 12B is a graph illustrating an example of a result of a change in tumor volume of a tumor model mouse.
Fig. 12C is a graph illustrating an example of a result of a change in tumor volume of a tumor model mouse.

### Description of Embodiments

Hereinafter, contents of the present invention will be described in detail. Description of constitution requirements described below may be made based on a representative embodiment of the present invention, but the present invention is not limited to such an embodiment.

Note that, in the present specification, "to" indicating a numerical range is used to mean that numerical values described before and after the numerical range are included as a lower limit value and an upper limit value.

In a stepwisely described numerical range in the present disclosure, an upper limit value or a lower limit value described in one numerical range may be replaced with an upper limit value or a lower limit value of another stepwisely described numerical range. In addition, in a numerical range described in the present disclosure, an upper limit value or a lower limit value of the numerical range may be replaced with a value described in Examples.

In addition, in the present disclosure, "% by mass", "% by weight", and "Wt%" have the same meaning, and "parts by mass" and "parts by weight" have the same meaning.

Furthermore, in the present disclosure, a combination of two or more preferred aspects is a more preferred aspect.

A polypeptide, a polynucleotide, a vector, and a cell provided by the present disclosure can be in an isolated state. That is, a polypeptide, a polynucleotide, a vector, and a cell described in the present specification can be an isolated polypeptide, an isolated polynucleotide, an isolated vector, and an isolated cell.

### (Anti-EGFRviii antibody)

An anti-EGFRviii antibody according to the present disclosure is an antibody or an antigen-binding fragment selected from the group consisting of the following (a-1) to (a-3).

The anti-EGFRviii antibody according to the present disclosure is an antibody or an antigen-binding fragment including CDR1, CDR2, and CDR3 in a heavy chain variable region (hereinafter, also referred to as "VH region") consisting of the following specific amino acid sequence, and CDR1, CDR2, and CDR3 in a light chain variable region (hereinafter, also referred to as "VL region") consisting of the following specific amino acid sequence, and having binding ability to EGFRviii, and thereby specifically binds to EGFRviii. The anti-EGFRviii antibody according to the present disclosure can also be used as a target antigen-binding region of a chimeric antigen receptor (CAR).

Examples of the anti-EGFRviii antibody include a monoclonal antibody and a polyclonal antibody, and a monoclonal antibody is preferable from a viewpoint of specificity to EGFRviii.

### <Antibody or antigen-binding fragment>

The anti-EGFRviii antibody according to the present disclosure is an antibody or an antigen-binding fragment selected from the group consisting of the following (a-1) to (a-3).

The antibody or the antigen-binding fragment is not particularly limited as long as it has binding ability to EGFRviii, and examples thereof include small molecule antibodies such as a single chain variable fragment(scFv) as described later, Fab, diabody, ScDb, scFv₂-Fc, IgG-scFv, scFv-HSA-scFv, Fab-scFv-Fc, Fab-scFv, and scFv-Fc.

### [(a-1) to (a-3)]

(a-1) an antibody or an antigen-binding fragment thereof including:
   a heavy chain variable region consisting of heavy chain variable regions CDR1, CDR2, and CDR3 each including the amino acid sequence set forth in SEQ ID NO: 1 or 4; and
   a light chain variable region consisting of light chain variable regions CDR1, CDR2, and CDR3 each including the amino acid sequence set forth in SEQ ID NO: 2, 3, 5, or 6, and
   having binding ability to EGFRviii.
(a-2) an antibody or an antigen-binding fragment thereof including:
   a heavy chain variable region consisting of an amino acid sequence having one or a plurality of amino acids mutated from the amino acid sequence set forth in SEQ ID NO: 1 or 4; and
   a light chain variable region consisting of an amino acid sequence having one or a plurality of amino acids mutated from the amino acid sequence set forth in SEQ ID NO: 2, 3, 5, or 6, and
   having binding ability to EGFRviii; and
(a-3) an antibody or an antigen-binding fragment thereof including:
   a heavy chain variable region consisting of an amino acid sequence having 70% or more of sequence identity to the amino acid sequence set forth in SEQ ID NO: 1 or 4; and
   a light chain variable region consisting of an amino acid sequence having 70% or more of sequence identity to the amino acid sequence set forth in SEQ ID NO: 2, 3, 5, or 6;
      and
   having binding ability to EGFRviii.

As long as the heavy chain variable region defined in (a-1) includes the amino acid sequence of CDR1, the amino acid sequence of CDR2, and the amino acid sequence of CDR3 in the heavy chain variable region consisting of the amino acid sequence set forth in SEQ ID NO: 1 or 4, the heavy chain variable region defined in (a-1) may be different from the amino acid sequence set forth in SEQ ID NO: 1 or 4 in a portion other than these sequences. That is, the heavy chain variable region defined in (a-1) may be different from the heavy chain variable region consisting of the amino acid sequence set forth in SEQ ID NO: 1 or 4 in a portion other than the CDR1 region, the CDR2 region, and the CDR3 region in the heavy chain variable region. As long as the light chain variable region defined in (a-1) includes the amino acid sequence of CDR1, the amino acid sequence of CDR2, and the amino acid sequence of CDR3 in the light chain variable region consisting of the amino acid sequence set forth in SEQ ID NO: 2, 3, 5, or 6, the light chain variable region defined in (a-1) may be different from the amino acid sequence set forth in SEQ ID NO: 2, 3, 5, or 6 in a portion other than these sequences. That is, the light variable region defined in (a-1) may be different from the light chain variable region consisting of the amino acid sequence set forth in SEQ ID NO: 2, 3, 5, or 6 in a portion other than the CDR1 region, the CDR2 region, and the CDR3 region in the light chain variable region. The positions of mutations in the amino acid sequence set forth in SEQ ID NO: 1 or 4 and the amino acid sequence set forth in SEQ ID NO: 2, 3, 5, or 6 in (a-2) and (a-3) may be in regions other than CDR1, CDR2, and CDR3.

The "CDR" of CDR1, CDR2, and CDR3 refers to a region estimated to be a region in direct contact with an antigen in a variable region of an antibody molecule and including a complementarity determining region (CDR).

Note that CDR can be determined by any definition known as a definition of CDR, and for example, a definition by Kabat, Chothia, AbM, contact, IMGT, Aho, or Mrtin (Enhanced Chothia) can be used. The amino acid sequence of CDR of the anti-EGFRviii antibody according to the present disclosure may be defined by any of these.

### -Amino acid sequences of VH regions CDRs 1 to 3 defined by Kabat-

As examples of VH regions CDRs 1 to 3 each including the amino acid sequence set forth in SEQ ID NO: 1 or 4,
amino acid sequences of CDRs 1 to 3 defined by Kabat are represented by SEQ ID NO: 31 to 33, respectively.

### -Amino acid sequences of VL regions CDRs 1 to 3 defined by Kabat-

As an example of a VL region CDR1 including the amino acid sequence set forth in SEQ ID NO: 2, an amino acid sequence of CDR1 defined by Kabat is represented by SEQ ID NO: 37.

As an example of a VL region CDR1 including the amino acid sequence set forth in SEQ ID NO: 3, 5, or 6, an amino acid sequence of CDR1 defined by Kabat is represented by SEQ ID NO: 34.

As examples of VL regions CDR2 and CDR3 each including the amino acid sequence set forth in SEQ ID NO: 2, 3, 5, or 6, amino acid sequences of CDR2 and CDR3 defined by Kabat are represented by SEQ ID NO: 35 and 36, respectively.

In (a-1) to (a-3), the heavy chain variable region including the heavy chain variable regions CDR1, CDR2, and CDR3 is preferably SEQ ID NO: 4.

In (a-1) to (a-3), the light chain variable region including the light chain variable regions CDR1, CDR2, and CDR3 is preferably SEQ ID NO: 5 or 6.

In the above (a-2), "a plurality" may be, for example, 2 to 30, is preferably 2 to 20, more preferably 2 to 10, and still more preferably 2 to 5, and may be, for example, 2, 3, 4, or 5. The "mutation" may be any of deletion, substitution, addition, and insertion, and may be a combination thereof.

The position of the mutation is preferably a region other than CDR1 to CDR3 (that is, a framework region).

A framework sequence of a human antibody can be selected from, for example, a framework sequence of an amino acid sequence of a human antibody registered in a known sequence database such as GenBank, and an amino acid sequence selected from common sequences (Human Most Homologous Consensus Sequence; Kabat, E.A., et al., Sequences of Proteins of Immunological Interest, US Dept.Health and HumanServices, 1991) derived from subgroups of a human antibody.

In the above (a-3), the sequence identity is not particularly limited as long as it is 70% or more, but may be 80% or more, 900 or more, 950 or more, 960 or more, 970 or more, 980 or more, or 99% or more.

Note that the sequence identity (that is, homology) between amino acid sequences is determined by juxtaposing two amino acid sequences with a gap in a portion corresponding to insertion and deletion such that the largest number of corresponding amino acids match each other, and determining a ratio of matched amino acids to the entire amino acid sequence excluding the gap in the obtained alignment.

The sequence identity between amino acid sequences can be determined using various types of homology search software known in the present technical field. For example, a value of the sequence identity of the amino acid sequence can be obtained by calculation based on an alignment obtained by known homology search software BLASTP.

In the anti-EGFRviii antibody according to the present disclosure, the heavy chain variable region preferably has T at position 21, F at position 24, F at position 54, K at position 59, S at position 67, K at position 73, T or K at position 77, and K or T at position 83 in the amino acid sequence set forth in SEQ ID NO: 1 or 4 from a viewpoint of specifically binding to EGFRviii and also being able to be used as a target antigen-binding region of a chimeric antigen receptor CAR.

Note that, in the amino acid sequence set forth in SEQ ID NO: 1 or 4, T at position 21 means that an amino acid at a position corresponding to position 21 counted from an N-terminus side of the amino acid sequence set forth in SEQ ID NO: 1 or 4 is T at the time of alignment with the amino acid sequence set forth in SEQ ID NO: 1 or 4.

In the anti-EGFRviii antibody according to the present disclosure, the light chain variable region preferably has M at position 4, E or G at position 16, Q at position 27, K at position 35, S at position 55, N at position 58, R at position 74, K at position 79, S at position 81, D at position 87, and V at position 99 in the amino acid sequence set forth in SEQ ID NO: 2, 3, 5, or 6 from a viewpoint of specifically binding to EGFRviii and also being able to be used as a target antigen-binding region of a chimeric antigen receptor.

Note that, in the present specification, an amino acid or an amino acid residue may be represented by one alphabetical letter or three alphabetical letters.

### [SG sequence]

The anti-EGFRviii antibody preferably has an Asn-Gly (NG) sequence in the amino acid sequence converted into a Ser-Gly (SG) sequence, more preferably has an NG sequence in the light chain variable region converted into an SG sequence, and still more preferably has an NG sequence of CDR1 in the light chain variable region converted into an SG sequence from a viewpoint of maintaining binding performance to EGFRviii.

Examples of the amino acid sequence obtained by converting the NG sequence to the SG sequence include the amino acid sequence set forth in SEQ ID NO: 3, 5, or 6.

### [Human antibody]

An organism from which the anti-EGFRviii antibody is derived is not particularly limited, and may be an antibody derived from an animal such as a human, a mouse, a rat, a hamster, a rabbit, or a monkey, but a human antibody is preferable.

In addition, the anti-EGFRviii antibody according to the present disclosure includes not only a monoclonal antibody but also an artificially modified antibody such as a chimeric antibody, a humanized antibody, or a bispecific antibody.

The anti-EGFRviii antibody is preferably a humanized antibody from a viewpoint of clinical utility. A method for producing a humanized antibody is not particularly limited, and the humanized antibody can be produced using a known method. Examples thereof include a production method in which an antigen binding site of an antibody produced with a mouse using genetic engineering is transplanted to a human-derived antibody molecule.

Examples of the humanized antibody (human anti-EGFRviii antibody) include an antibody having the amino acid sequence set forth in SEQ ID NO: 4 as a heavy chain variable (VH) region and the amino acid sequence set forth in SEQ ID NO: 5 or 6 as a light chain variable (VL) region.

A method for producing the anti-EGFRviii antibody according to the present disclosure is not particularly limited, and a known antibody producing method can be used. For example, a method can be used in which a nucleic acid having a sequence encoding the anti-EGFRviii antibody is inserted into an expression vector, and the expression vector is introduced into a host cell and expressed.

### <Single chain variable fragment (scFv)>

The anti-EGFRviii antibody according to the present disclosure may be a single chain variable fragment (scFv).

The scFv is a polypeptide in which a heavy chain variable region (VH region) of an antibody and a light chain variable region (VL region) of the antibody are linked to each other with a peptide linker. The scFv is generally used as a target antigen-binding region of a chimeric antigen receptor (CAR) described later.

In the scFv, the order in which the VH region and the VL region are linked to each other with a peptide linker is not particularly limited, and may be the order of the VH region, the peptide linker, and the VL region, or the order of the VL region, the peptide linker, and the VH region from an N-terminus side.

In an embodiment of the present disclosure, the scFv preferably includes a light chain variable region of the antibody, a peptide linker linking a heavy chain variable region and the light chain variable region to each other, and the heavy chain variable region sequentially from an N-terminus.

In the scFv, the peptide linker linking the VH region and the VL region to each other is not particularly limited, and it is only required to use those generally used for the scFv.

Examples of the peptide linker include linker 15 (SEQ ID NO: 41) and linker 25 (SEQ ID NO: 42), but are not limited thereto.

In a case where the anti-EGFRviii antibody is an scFv, the scFv may be a polypeptide selected from selected from the group consisting of the following (I) to (III).
(I) a polypeptide including an amino acid sequence selected from the group consisting of SEQ ID NOs: 12 to 18 and 38;
(II) a polypeptide including an amino acid sequence having 70% or more (preferably 80% or more, more preferably 90% or more, still more preferably 95% or more, further still more preferably 970 or more) of sequence identity to the amino acid sequence selected from the group consisting of SEQ ID NOs: 12 to 18 and 38, and having binding ability to EGFRviii; and
(III) a polypeptide including an amino acid sequence having one or a plurality of amino acids mutated from the amino acid sequence selected from the group consisting of SEQ ID NOs: 12 to 18 and 38, and having binding ability to EGFRviii.

The sequence identity in the above (II) is synonymous with the sequence identity in the above (a-3), and a preferred aspect of the sequence identity in the above (II) is also similar to a preferred aspect of the sequence identity in the above (a-3).

"A plurality" in the above (III) is synonymous with "a plurality" in the above (a-2), and a preferred aspect of "a plurality" in the above (III) is also similar to a preferred aspect of "a plurality" in the above (a-2).

The scFv is preferably
(I) a polypeptide including the amino acid sequence set forth in SEQ ID NO: 13, 15, or 17;
(II) a polypeptide consisting of an amino acid sequence having 70% or more (preferably 80% or more, more preferably 90% or more, still more preferably 95% or more, further still more preferably 970 or more) of sequence identity to the amino acid sequence set forth in SEQ ID NO: 13, 15, or 17, and having binding ability to EGFRviii; or
(III) a polypeptide consisting of an amino acid sequence having one or a plurality of amino acids mutated from the amino acid sequence set forth in SEQ ID NO: 13, 15, or 17, and having binding ability to EGFRviii, and more preferably

(I) a polypeptide including the amino acid sequence set forth in SEQ ID NO: 15, or 17;
(II) a polypeptide consisting of an amino acid sequence having 70% or more (preferably 80% or more, more preferably 90% or more, still more preferably 95% or more, further still more preferably 970 or more) of sequence identity to the amino acid sequence set forth in SEQ ID NO: 15, or 17, and having binding ability to EGFRviii; or
(III) a polypeptide consisting of an amino acid sequence having one or a plurality of amino acids mutated from the amino acid sequence set forth in SEQ ID NO: 15, or 17, and having binding ability to EGFRviii.

### (Polypeptide)

A polypeptide according to the present disclosure preferably includes a target antigen-binding region, a transmembrane region, and a signal transduction region, in which the target antigen-binding region is the anti-EGFRviii antibody.

In an aspect of the present disclosure, the polypeptide according to the present disclosure is preferably a chimeric antigen receptor (CAR).

In the present specification, the "chimeric antigen receptor (CAR)" means an artificial chimeric protein in which a portion derived from an antibody that specifically recognizes an antigen is fused with a signal transduction region that induces activation of immune cells.

The chimeric antigen receptor is preferably a chimeric protein including a target antigen-binding region, a transmembrane region, and a signal transduction region. The target antigen-binding region preferably includes the anti-EGFRviii antibody according to the present disclosure.

In a case where the polypeptide according to the present disclosure is a CAR, and the target antigen-binding region included in the polypeptide is an anti-EGFRviii antibody, the polypeptide may be referred to as an anti-EGFRviiiCAR.

Note that the chimeric protein means a protein including a sequence derived from two or more different types of proteins.

The CAR is not limited to those including only the above three regions, and examples of the CAR also include those further including another region.

Hereinafter, details of each region included in the polypeptide according to the present disclosure will be described.

### <Target antigen-binding region>

The "target antigen-binding region" means a region that binds to a target antigen and is located outside a cell in the polypeptide according to the present disclosure. Note that details of the cell will be described later.

For example, a CAR expressed in a T cell into which a chimeric antigen receptor (CAR) gene has been introduced (hereinafter, also referred to as "CAR-T cell") migrates to a cell membrane, and to be in state in which a target antigen-binding region located outside the cell and a T cell activation signal transduction region located in the cell are linked to each other via a transmembrane region penetrating the cell membrane. When the CAR-T cell comes into contact with a cell having a target antigen as a membrane antigen, the target antigen-binding region binds to the target antigen, whereby a T cell activation signal is transmitted from the T cell activation signal transduction region into the T cell, and the T cell is activated. Also in a case where the polypeptide according to the present disclosure is expressed in a cell other than the T cell, the cell can be activated by a similar method or an equivalent method.

### [EGFRviii]

In the polypeptide according to the present disclosure, the target antigen to which the target antigen-binding region binds is EGFRviii.

EGFRviii is one of variants of an epidermal growth factor receptor (EGFR) and is considered not to be expressed in a normal cell.

EGFRviii is known to be expressed in glioblastoma multiforme, glioma, anaplastic astrocytoma, medulloblastoma, breast cancer, non-small cell lung cancer, ovarian cancer, pancreatic cancer, renal cell cancer, bladder cancer, prostate cancer, colorectal cancer, head and neck cancer, central nervous system cancer, skin cancer, and metastatic cancers thereof, and is one of target markers for cancer therapy.

The target antigen-binding region of the polypeptide according to the present disclosure includes the anti-EGFRviii antibody according to the present disclosure. Therefore, the target antigen-binding region includes CDR described in the description of the anti-EGFRviii antibody.

Note that CDR can be determined by any definition known as a definition of CDR, and for example, a definition by Kabat, Chothia, AbM, contact, IMGT, Aho, or Mrtin (Enhanced Chothia) can be used.

As the target antigen-binding region, a polypeptide containing a single chain variable fragment (scFv) of the anti-EGFRviii antibody is a preferred example of the target antigen-binding region. In a case of a bispecific antibody having two target antigen-binding regions that bind to different antigens, one of the target antigen-binding regions may be the anti-EGFRviii antibody.

The single chain variable fragment (scFv) has the same meaning as the scFv in the anti-EGFRviii antibody, and a preferred aspect of the single chain variable fragment (scFv) is also similar to a preferred aspect of the scFv in the anti-EGFRviii antibody.

### <Transmembrane region>

The polypeptide according to the present disclosure preferably includes a transmembrane region.

In the present specification, the "transmembrane region" means a region that penetrates a cell membrane and links an extracellular region and an intracellular region to each other when the polypeptide according to the present disclosure is expressed in a cell.

The transmembrane region is not particularly limited as long as it is a polypeptide having a function of penetrating a cell membrane. The transmembrane region may be derived from a natural protein or may be artificially designed.

A transmembrane region derived from a natural protein can be obtained from any membrane-bound protein or any transmembrane protein. The transmembrane region preferably transmits an activation signal to a T cell activation signal transduction region corresponding to binding of a target antigen to a target antigen-binding region.

The transmembrane region is not particularly limited, and examples thereof include transmembrane regions such as an a chain and a β chain of a T cell receptor, CD3ζ, CD28, CD3ε, CD45, CD4, CD5, CD8, CD9, CD16, CD22, CD33, CD37, CD64, CD80, CD86, CD134, CD137, ICOS, CD154, and GITR.

Among these, the CD8 transmembrane region is preferable.

An organism from which a protein in the transmembrane region is derived is not particularly limited, and may be a human, a mouse, a rat, a hamster, a rabbit, or a monkey, but a human is preferable. Amino acid sequences of these proteins are available from a known sequence database such as GenBank. Examples of an amino acid sequence of human CD8 include an amino acid sequence registered as GenBank number: NM_001768.6. Examples of an amino acid sequence of the transmembrane region include the amino acid sequences set forth in SEQ ID NOs: 19 and 20.

The transmembrane region may be a variant of a transmembrane region derived from a natural protein as described above.

Examples of the variant of a transmembrane region derived from a natural protein include the following:
(2a) A polypeptide consisting of an amino acid sequence having 70% or more of sequence identity (homology) with an amino acid sequence of a transmembrane region derived from a natural protein (for example, SEQ ID NOs: 19 and 20), and having transmembrane ability; and
(2b) A polypeptide consisting of an amino acid sequence having one or a plurality of amino acids mutated from an amino acid sequence of a transmembrane region derived from a natural protein (for example, SEQ ID NOs: 19 and 20), and having transmembrane ability.

In the amino acid sequence (2a) of the transmembrane region, the sequence identity is not particularly limited as long as it is 70% or more, but is preferably 80% or more, more preferably 85% or more, still more preferably 90% or more, and particularly preferably 95% or more.

In the amino acid sequence (2b) of the transmembrane region, "a plurality" may be, for example, 2 to 10, and is preferably 2 to 5, more preferably 2 to 4, and still more preferably 2 or 3. The "mutation" may be any of deletion, substitution, addition, and insertion, and may be a combination thereof.

### <Signal transduction region>

The polypeptide according to the present disclosure preferably includes a signal transduction region. The signal transduction region only needs to be a region that can transmit a signal into a cell expressing the polypeptide according to the present disclosure when the target antigen-binding site (that is, anti-EGFRviii antibody) binds to EGFRviii that is a target antigen. Note that details of the cell will be described later.

As an example, in a case where a T cell is used as the cell, the signal transduction region is preferably a T cell activation signal transduction region.

In the present specification, the "signal transduction region" means a region that is located in a cell and transmits a signal into the cell when the polypeptide according to the present disclosure is expressed in the cell.

In the present specification, the "signal transduction region" means a region that is located in a cell and transmits an activation signal of the cell into the cell when the polypeptide according to the present disclosure is expressed in the cell.

In a T cell, when a major histocompatibility complex (MHC)-peptide complex binds to a T cell receptor (TCR), a T cell activation signal is transmitted into the cell via a TCR-CD3 complex, and various phosphorylation signals are induced (primary signal transduction). In a CAR-T cell, a primary signal can be transmitted to the T cell without depending on an interaction with MHC by binding of an antigen to a target antigen-binding site.

In addition, it is known that a costimulatory molecule expressed on a cell surface of a T cell (for example, CD28 or 4-1BB described later) transmits a costimulatory signal into a cell by binding to a ligand specific to each costimulatory molecule expressed on a cell surface of an antigen-presenting cell, and assists activation of the T cell (secondary signal transduction). In the CAR-T cell, by incorporating such a costimulatory molecule into the CAR, a secondary signal can be transmitted to the T cell when a target antigen-binding site binds to an antigen.

In the present specification, the "T cell activation signal transduction" includes both the primary signal transduction and the secondary signal transduction. In addition, the "T cell activation signal transduction region" means an intracellular region of a protein involved in at least one of the primary signal transduction and the secondary signal transduction, involved in the signal transduction.

The signal transduction region is not particularly limited as long as it is a cell activation signal transduction region of a protein involved in activation signal transduction of a cell.

For example, it is known that an immunoreceptor tyrosine-based activation motif (ITAM) is involved in the primary signal transduction. Therefore, examples of the signal transduction region include a cell activation signal transduction region of a protein having an ITAM.

Examples of the protein having an ITAM include CD3ζ, FcRγ, FcRβ, CD3γ, CD35, CD3ε, CD5, CD22, CD79a, CD79b, and CD66d. The signal transduction region including an ITAM is a preferred example of the cell activation signal transduction region used in the polypeptide according to the present disclosure. More preferred examples thereof include a cell activation signal transduction region such as CD3ζ.

In addition, a costimulatory molecule is involved in the secondary signal transduction as described above. Therefore, examples of the signal transduction region also include a signal transduction region of a costimulatory molecule.

Examples of the costimulatory molecule include CD2, CD4, CD5, CD8, CD27, CD28, OX40 (CD134), 4-1BB (CD137), ICOS, CD154, herpesvirus entry mediator (HVEM), GITR, and FcReceptor-associated γchain. Cell activation signal transduction regions of these proteins are also preferred examples of the cell activation signal transduction region used in the polypeptides according to the present disclosure.

More preferred examples thereof include cell activation signal transduction regions of CD28 and 4-1BB.

An organism from which a protein in the signal transduction region is derived is not particularly limited, and may be a human, a mouse, a rat, a hamster, a rabbit, or a monkey, but a human is preferable. Note that amino acid sequences of these proteins are available from a known sequence database such as GenBank.

Examples of an amino acid sequence of human CD3ζ include an amino acid sequence registered as GenBank number: NM_000734.3, and examples of an amino acid sequence of the signal transduction region include the amino acid sequence set forth in SEQ ID NO: 25.

Examples of an amino acid sequence of human CD28 include an amino acid sequence registered as GenBank number: NM_006139.2, and examples of an amino acid sequence of the signal transduction region include the amino acid sequence set forth in SEQ ID NO: 26. Examples of an amino acid sequence of human 4-1BB include an amino acid sequence registered as GenBank number: NM_001561.5, and examples of an amino acid sequence of the signal transduction region include the amino acid sequence set forth in SEQ ID NO: 27.

The signal transduction region may be a variant of a signal transduction region derived from a natural protein as described above. Examples of the variant of an activation signal transduction region derived from a natural protein include the following:
(3a) A polypeptide consisting of an amino acid sequence having 70% or more of sequence identity (homology) with an amino acid sequence of a signal transduction region derived from a natural protein (for example, SEQ ID NOs: 25 to 27), and having cell activation signal transduction ability; and
(3b) A polypeptide consisting of an amino acid sequence having one or a plurality of amino acids mutated from an amino acid sequence of a signal transduction region derived from a natural protein (for example, SEQ ID NOs: 25 to 27), and having cell activation signal transduction ability.

In the amino acid sequence (3a) of the signal transduction region, the sequence identity is not particularly limited as long as it is 70% or more, but is preferably 80% or more, more preferably 85% or more, still more preferably 90% or more, and particularly preferably 95% or more.

In the above amino acid sequence (3b), "a plurality" may be, for example, 2 to 30, is preferably 2 to 20, more preferably 2 to 10, and still more preferably 2 to 5 in a case where a protein involved in the primary signal transduction is used. In addition, "a plurality" may be, for example, 2 to 15, and is preferably 2 to 10, more preferably 2 to 5, and still more preferably 2 or 3 in a case where a costimulatory molecule is used.

The "mutation" may be any of deletion, substitution, addition, and insertion, and may be a combination thereof.

The number of signal transduction regions included in the polypeptide according to the present disclosure is not limited to one, and a plurality of signal transduction regions can be included. In this case, the plurality of signal transduction regions may be the same signal transduction region or different signal transduction regions.

The polypeptide according to the present disclosure preferably includes two or more signal transduction regions. In this case, the signal transduction region included in the polypeptide according to the present disclosure is preferably a combination of a signal transduction region involved in the primary signal transduction and a signal transduction region involved in the secondary signal transduction.

Specific examples thereof include a combination of cell activation signal transduction regions of CD3 ζ and CD28, a combination of cell activation signal transduction regions of CD3ζ and 4-1BB, and a combination of cell activation signal transduction regions of CD3ζ, CD28, and 4-1BB.

Note that in a case where a signal transduction region involved in the primary signal transduction and a signal transduction region involved in the secondary signal transduction are combined, the signal transduction region involved in the primary signal transduction is preferably arranged on a C-terminus side.

In the above specific example, a T cell activation signal transduction region of CD3ζ is preferably arranged on a C-terminus side of a T cell activation signal transduction region of CD28 or 4-1BB. In a case where both CD28 and 4-1BB are used, CD28 and 4-1BB may be arranged in any order, but for example, CD28 and 4-1BB may be arranged in this order from an N-terminus side.

In a case where only one T cell activation signal transduction region is used, a T cell activation signal transduction region involved in the primary signal transduction is preferably used, and a T cell activation signal transduction region of CD3ζ is more preferably used.

### [Other region]

The polypeptide according to the present disclosure may include a region other than the transmembrane region and the signal transduction region (hereinafter, also referred to as "other region"). Examples of the other region include an extracellular hinge region, a cytoplasmic region, a spacer region, and a signal peptide.

### -Extracellular hinge region-

The polypeptide according to the present disclosure may include an extracellular hinge region.

In the present specification, the "extracellular hinge region" means a region linking an extracellular target antigen-binding region and a transmembrane region to each other. The extracellular hinge region may be a part of the transmembrane region.

The extracellular hinge region is not particularly limited as long as it can link the target antigen-binding region and the transmembrane region to each other. A protein constituting the extracellular hinge region may be derived from a natural protein or may be artificially designed.

The extracellular hinge region can include, for example, about 1 to 100 amino acids, preferably about 10 to 70 amino acids. The extracellular hinge region preferably does not interfere with EGFRviii binding ability of the target antigen-binding region and does not interfere with signal transduction by the signal transduction region.

Examples of the extracellular hinge region include extracellular hinge regions of CD8, CD28, and CD4. As the extracellular hinge region, a hinge region of an immunoglobulin (for example, IgG4) may be used. The extracellular hinge region is preferably an extracellular hinge region of CD8.

An organism from which a protein in the extracellular hinge region is derived is not particularly limited, and may be a human, a mouse, a rat, a hamster, a rabbit, or a monkey, but a human is preferable. Note that amino acid sequences of these proteins constituting the extracellular hinge region are available from a known sequence database such as GenBank. Examples of the amino acid sequence of human CD8 include those described above.

Examples of an amino acid sequence of the extracellular hinge region include the amino acid sequences set forth in SEQ ID NOs: 21 and 22.

The extracellular hinge region may be a variant of an extracellular hinge region derived from a natural protein as described above. Examples of the variant of an extracellular hinge region derived from a natural protein include the following:
(4a) A polypeptide consisting of an amino acid sequence having 70% or more of sequence identity (homology) with an amino acid sequence of an extracellular hinge region derived from a natural protein (for example, SEQ ID NOs: 21 and 22); and
(4b) A polypeptide consisting of an amino acid sequence having one or a plurality of amino acids mutated from an amino acid sequence of an extracellular hinge region derived from a natural protein (for example, SEQ ID NOs: 21 and 22).

In the amino acid sequence (4a) of the extracellular hinge region, the sequence identity is not particularly limited as long as it is 70% or more, but is preferably 80% or more, more preferably 85% or more, still more preferably 90% or more, and particularly preferably 95% or more.

In the amino acid sequence (4b) of the extracellular hinge region, "a plurality" may be, for example, 2 to 20, and is preferably 2 to 15, more preferably 2 to 10, and still more preferably 2 to 5. The "mutation" may be any of deletion, substitution, addition, and insertion, and may be a combination thereof.

### -Spacer region-

In the polypeptide according to the present disclosure, regions such as the target antigen-binding region, the transmembrane region, and the signal transduction region may be linked to each other via a spacer region.

In the present specification, the "spacer region" refers to a short peptide linking two functional regions (domains) of a protein to each other. The spacer region is not particularly limited, and it is only required to use those generally used for producing a chimeric protein.

The length of the spacer region is 1 to 100 amino acids, and preferably 10 to 50 amino acids. Examples of the spacer region include a glycine-serine continuous sequence.

### -Signal peptide-

The polypeptide according to the present disclosure may contain a signal peptide.

In the present specification, the "signal peptide" refers to a peptide that indicates localization of a membrane protein or a secreted protein. The signal peptide is generally a peptide containing about 5 to 60 amino acids present at an N-terminus of a membrane protein, and a signal peptide has been removed in a mature protein in which localization has been completed.

The signal peptide contained in the polypeptide according to the present disclosure is preferably a signal peptide that indicates localization to a cell membrane, and preferably a signal peptide of a membrane protein.

Examples of the signal peptide include signal peptides such as an a chain and a β chain of a T cell receptor, CD3ζ, CD28, CD3ε, CD45, CD4, CD5, CD8, CD9, CD16, CD22, CD33, CD37, CD64, CD80, CD86, CD134, CD137, ICOS, CD154, GITR, an immunoglobulin heavy chain, and an immunoglobulin light chain.

Specific examples of an amino acid sequence of the signal peptide include the amino acid sequences set forth in SEQ ID NOs: 23 and 24.

In the polypeptide according to the present disclosure, the above regions are preferably arranged in the order of the target antigen-binding region, the transmembrane region, and the signal transduction region from an N-terminus. These regions may be directly linked to each other, or may be linked to each other via another region, a spacer sequence, or the like.

In a case where the polypeptide according to the present disclosure includes an extracellular hinge region, the extracellular hinge region is preferably arranged between the target antigen-binding region and the transmembrane region.

In a case where the polypeptide according to the present disclosure contains a signal peptide, the signal peptide is preferably arranged at an N-terminus of the polypeptide.

Specific examples of the polypeptide according to the present disclosure include a polypeptide including a target antigen-binding region containing an scFv of an anti-EGFRviii antibody, a CD8 transmembrane region (or a variant thereof), a CD28 cell activation signal transduction region (or a variant thereof), a 4-1BB T cell activation signal transduction region (or a variant thereof), and a CD3ζ cell activation signal transduction region (or a variant thereof).

Other preferred examples thereof include a CAR including a target antigen-binding region containing an scFv of an anti-EGFRviii antibody, a CD8 extracellular hinge region (or a variant thereof), a CD8 transmembrane region (or a variant thereof), a CD28 cell activation signal transduction region (or a variant thereof), a 4-1BB T cell activation signal transduction region (or a variant thereof), and a CD3ζ cell activation signal transduction region (or a variant thereof).

Other preferred examples thereof include a CAR including a target antigen-binding region containing an scFv of an anti-EGFRviii antibody, a CD8 extracellular hinge region (or a variant thereof), a CD8 transmembrane region (or a variant thereof), a 4-1BB cell activation signal transduction region (or a variant thereof), and a CD3ζ cell activation signal transduction region (or a variant thereof).

Examples of the polypeptide (preferably CAR) according to the present disclosure include a CAR containing an scFv including an amino acid sequence selected from the group consisting of SEQ ID NOs: 12 to 18 and 38.

### (Cell)

A cell according to the present disclosure is preferably a cell expressing the polypeptide according to the present disclosure.

The cell according to the present disclosure preferably expresses the polypeptide (hereinafter, also referred to as "anti-EGFRviiiCAR" for convenience, but the polypeptide is not limited to the CAR).

In a cell expressing EGFRviii (EGFRviii-expressing cell), EGFRviii is abundantly present in a cell surface layer. It is estimated that when the cell according to the present disclosure comes into contact with a cell expressing EGFRviii, the cell according to the present disclosure binds to EGFRviii in a surface layer of the EGFRviii-expressing cell due to a target antigen-binding region of the anti-EGFRviiiCAR, whereby the cell according to the present disclosure is activated to release cytotoxic granules and produce a cytokine, and the cytotoxic granules and the cytokine destroy the EGFRviii-expressing cell.

The cell according to the present disclosure is preferably a mammalian cell. The mammalian cell may be, for example, a human cell or a cell of a non-human mammal such as a mouse, a rat, a cow, a sheep, a horse, a dog, a pig, or a monkey. Among these, the mammalian cell is more preferably a human cell.

The type of cell is not particularly limited, and a cell that can reach a cancer tissue through a blood vessel can be widely used. Example thereof include a cell collected from blood, bone marrow fluid, spleen, thymus, lymph node, or the like, an immune cell that infiltrates a cancer tissue such as a primary tumor, a metastatic tumor, or cancerous ascites, and a mesenchymal stem cell.

In the present disclosure, the "immune cell" means a cell that performs an immune function in a living body. The immune cell may also be referred to as an immunocompetent cell. The immune cell is preferably a cell separated from a living body. Examples of the immune cell include a lymphocyte such as a T cell, a natural killer cell (NK cell), or a B cell, an antigen-presenting cell such as a monocyte, a macrophage, or a dendritic cell, and a granulocyte such as a neutrophil, an eosinophil, a basophil, or a mast cell.

More specifically, a peripheral blood mononuclear cell and the like separated from peripheral blood can be suitably used.

Among cells contained in the peripheral blood mononuclear cell, an effector cell is preferable, and as the effector cell, for example, a T cell and a precursor cell thereof are used. The type of T cell is not particularly limited, and may be any T cell such as an αβT cell, a γδT cell, a CD8 positive T cell, a cytotoxic T cell, a CD4 positive T cell, a helper T cell, a memory T cell, a naive T cell, a tumor-infiltrating T cell, or a natural killer T cell.

Among these, the T cell is more preferably a CD8 positive T cell or a cytotoxic T cell.

### «IL-7 and CCL19»

The cell according to the present disclosure preferably further expresses at least one of interleukin (IL)-7 (IL-7) and chemokine (C-C motif) ligand 19 (CCL19) in addition to an anti-EGFRviiiCAR.

The cell according to the present disclosure is preferably a cell expressing (i) an anti-EGFRviiiCAR and (ii) at least one of IL-7 and CCL19, and more preferably a cell expressing an anti-EGFRviiiCAR, IL-7, and CCL19. The IL-7 and/or CCL19 are preferably exogenous, that is, expressed from an exogenous nucleic acid.

IL-7 is an essential cytokine for T cell survival, and is produced by a non-hematopoietic cell such as a stromal cell of bone marrow, thymus, and lymphatic organs/tissues, but production in the T cell is hardly observed.

Meanwhile, CCL19 is produced mainly from a dendritic cell and a macrophage in the lymph node, and has a function of inducing migration of a T cell, a B cell, and a mature dendritic cell via a CC chemokine (C-C motif) receptor 7 (CCR7) which is a receptor of CCL19.

An organism from which each of IL-7 and CCL19 is derived is not particularly limited, and may be a human, a mouse, a rat, a hamster, a rabbit, or a monkey, but a human is preferable. Note that amino acid sequences of these proteins are available from a known sequence database such as GenBank.

Examples of an amino acid sequence of human IL-7 include an amino acid sequence registered as GenBank number: NM_000880.3 (SEQ ID NO: 28). Examples of an amino acid sequence of human CCL19 include an amino acid sequence registered as GenBank number: NM_006274.2 (SEQ ID NO: 29).

Note that IL-7 and CCL19 each contain a signal peptide, and the signal peptide is removed in a mature protein.

For example, in the amino acid sequence of human IL-7 set forth in SEQ ID NO: 28, a sequence of positions 1 to 25 corresponds to a signal peptide. In addition, for example, in the amino acid sequence of human CCL19 set forth in SEQ ID NO: 29, a sequence of positions 1 to 21 corresponds to a signal peptide.

In addition, IL-7 and CCL19 may be each a variant of a natural protein as described above. Examples of the variant of IL-7 include the following:
(6a) A polypeptide consisting of an amino acid sequence having 70% or more of sequence identity (homology) with an amino acid sequence of natural IL-7 (for example, SEQ ID NO: 28), and having a T cell immune function promoting function; and
(6b) A polypeptide consisting of an amino acid sequence having one or a plurality of amino acids mutated from an amino acid sequence of natural IL-7 (for example, SEQ ID NO: 28), and having a T cell immune function promoting function.

Examples of a variant of CCL19 include the following.

(7a) A polypeptide consisting of an amino acid sequence having 70% or more of sequence identity (homology) with an amino acid sequence of natural CCL19 (for example, SEQ ID NO: 29), and having a T cell immune function promoting function.

(7b) A polypeptide consisting of an amino acid sequence having one or a plurality of amino acids mutated from an amino acid sequence of natural CCL19 (for example, SEQ ID NO: 29), and having a T cell immune function promoting function.

Note that the "T cell immune function promoting function" means a function of maintaining and/or promoting survival, proliferation, cytotoxic activity, migration activity, infiltration activity into tumor tissue, and the like of a T cell.

In the above (6a) and (7a), the sequence identity is not particularly limited as long as it is 700 or more, but is preferably 800 or more, more preferably 850 or more, still more preferably 90% or more, and particularly preferably 950 or more.

In addition, in the above (6b) and (7b), "a plurality" may be, for example, 2 to 30, and is preferably 2 to 20, more preferably 2 to 10, and still more preferably 2 to 5.

The "mutation" may be any of deletion, substitution, addition, and insertion, and may be a combination thereof.

In addition, the variants of IL-7 and CCL19 may be obtained by changing signal peptides of these proteins to other signal peptides, or may be obtained by removing the signal peptides. Preferably, the variants of IL-7 and CCL19 each contain a signal peptide of a secreted protein and are secreted to the outside of a cell.

In a case where the cell according to the present disclosure is an isolated cell, by expressing at least one selected from the group consisting of IL-7 and CCL19, preferably from an exogenous nucleic acid, together with an anti-EGFRviiiCAR, the T cell immune function is promoted, and the cell can be a cell excellent in cytotoxic activity against an EGFRviii-expressing cell, infiltration into a tumor tissue, or viability in a tumor microenvironment.

The cell according to the present disclosure, preferably the immune cell, may further express another immune function control factor such as IL-15, CCL 21, IL-2, IL-4, IL-12, IL-13, IL-17, IL-18, IP -10, Interferon-γ, MIP-1alpha, GM-CSF, M-CSF, TGF-beta, or TNF-alpha, and the other immune control factor is preferably an immune function control factor other than IL-12. Note that these other immune control factors may be expressed from endogenous nucleic acids encoding the respective factors, or may be expressed from exogenous nucleic acids encoding the respective factors.

In general, in a case where an exogenous gene is introduced into a cell and forcibly expressed, an expression ratio tends to decrease as the number of exogenous genes increases. Therefore, in a case where IL-7, CCL19, and the like are forcibly expressed in addition to the anti-EGFRviiiCAR, there is a concern that an expression ratio of the anti-EGFRviiiCAR decreases. However, as described in Examples described later, when an anti-EGFRviii antibody selected from the group consisting of the above (a-1) to (a-3) is used, the anti-EGFRviiiCAR can be expressed at a sufficiently high ratio. The sufficiently high expression ratio indicates, for example, an expression ratio of 200 or more, 250 or more, or 300 or more.

In addition, the cell according to the present disclosure may express a suicide gene in addition to the anti-EGFRviiiCAR. Since the cell according to the present disclosure expresses a suicide gene, apoptosis can be induced in the cell according to the present disclosure as necessary.

The suicide gene is not particularly limited, and a known suicide gene can be used. Examples of the suicide gene include a thymidine kinase (HSV-TK) gene of herpes simplex virus and an inducible caspase 9 gene. A cell expressing HSV-TK can induce cell death by coexisting ganciclovir.

In addition, a cell expressing inducible caspase 9 can induce cell death by coexisting a chemical induction of dimerization (CID) such as AP1903.

An amino acid sequence of the suicide gene is available from a known sequence database such as GenBank. In addition, a sequence such as a vector containing a commercially available suicide gene can also be used.

The cell according to the present disclosure preferably contains an anti-EGFRviiiCAR.

The cell according to the present disclosure is preferably a cell containing an anti-EGFRviiiCAR and at least one selected from the group consisting of IL-7 and CCL19, more preferably a cell containing an anti-EGFRviiiCAR, IL-7, and CCL19, and may be a cell containing an anti-EGFRviiiCAR, IL-7, CCL19, and a suicide gene. Each of the IL-7 and CCL19 is preferably expressed from an exogenous nucleic acid (such as a vector introduced from the outside of a cell) encoding the IL-7 and CCL19 themselves.

The cell according to the present disclosure can be obtained by introducing a polynucleotide or a vector including a nucleotide sequence encoding an anti-EGFRviiiCAR described later into a cell.

### (Polynucleotide)

The polynucleotide according to the present disclosure preferably includes a nucleotide sequence encoding the polypeptide.

The polynucleotide according to the present disclosure is not particularly limited as long as it includes a nucleotide sequence encoding an anti-EGFRviiiCAR. The anti-EGFRviiiCAR is as described in the above section of [Chimeric antigen receptor (anti-EGFRviiiCAR)] .

The polynucleotide according to the present disclosure preferably includes a nucleotide sequence encoding the amino acid sequence of the anti-EGFRviiiCAR exemplified in the above section of [Chimeric antigen receptor (anti-EGFRviiiCAR)].

### <Nucleotide sequence>

Examples of a nucleotide sequence encoding the target antigen-binding region include a nucleotide sequence encoding an scFv of an anti-EGFRviii antibody.

More specific examples thereof include a nucleotide sequence encoding the amino acid sequence set forth in SEQ ID NO: 1 or 4 and a nucleotide sequence encoding the amino acid sequence set forth in SEQ ID NO: 2, 3, 5, or 6.

Examples of the nucleotide sequence encoding the amino acid sequence set forth in SEQ ID NO: 4 include the nucleotide sequence set forth in SEQ ID NO: 8. Examples of the nucleotide sequence encoding the amino acid sequence set forth in SEQ ID NO: 6 include the nucleotide sequence set forth in SEQ ID NO: 7.

The nucleotide sequence encoding an scFv of an anti-EGFRviii antibody is preferably linked by a nucleotide sequence encoding a peptide linker. The peptide linker is as described in the above section of "Chimeric antigen receptor". Examples of a nucleotide sequence encoding the linker 15 exemplified above include the nucleotide sequence set forth in SEQ ID NO: 43. Examples of a nucleotide sequence encoding the linker 25 include the nucleotide sequence set forth in SEQ ID NO: 44.

Specific examples of the nucleotide sequence encoding an scFv of an anti-EGFRviii antibody include the nucleotide sequences set forth in SEQ ID NOs: 9 and 10.

The nucleotide sequence encoding the target antigen-binding region is preferably codon-optimized according to the species of a cell to be introduced, and is preferably optimized to a human codon in a case of introduction into a human cell.

In addition, nucleotide sequences encoding the transmembrane region and the signal transduction region are available from a known sequence database such as GenBank. In addition, in a case where the polypeptide (preferably EGFRviiiCAR) includes another region such as an extracellular hinge region, a nucleotide sequence encoding the other region is also available from a known sequence database such as GenBank.

For example, in a case where a transmembrane region of human CD8 is used as the transmembrane region, examples of a nucleotide sequence encoding human CD8 include an amino acid sequence registered as GenBank No.: NM_001768.6, and examples of a nucleotide sequence encoding the transmembrane region include the nucleotide sequence set forth in SEQ ID NO: 45.

In addition, for example, in a case where T cell activation signal transduction regions of human CD3ζ, human CD28, and human 4-1BB are used as the T cell activation signal transduction region, examples of nucleotide sequences encoding human CD3ζ, human CD28, and human 4-1BB include amino acid sequences registered as GenBank numbers: NM_000734.3, NM_006139.2, and NM_001561.5, respectively, and examples of nucleotide sequences encoding the T cell activation signal transduction regions of human CD3ζ, human CD28, and human 4-1BB include the nucleotide sequence set forth in SEQ ID NOs: 46 to 48, respectively.

The nucleotide sequence encoding each of the above regions is not limited to a known one, and may be any sequence as long as it is a nucleotide sequence encoding each of the above regions. Due to degeneracy of a genetic code, there is a plurality of codons corresponding to one amino acid. Therefore, there are many nucleotide sequences encoding the same amino acid sequence. The nucleotide sequence encoding each of the above regions may be any of a plurality of nucleotide sequences generated by degeneracy of a genetic code as long as it encodes these regions.

The nucleotide sequence encoding each of the above regions is preferably codon-optimized according to the species of a cell to be introduced, and is preferably optimized to a human codon in a case of introduction into a human cell.

In addition, the nucleotide sequence encoding each of the regions may encode a variant of each of the regions derived from a natural protein. The variant of each of the regions is as described above in the above section of [Chimeric antigen receptor (anti-EGFRviiiCAR)].

In the nucleotide sequence encoding each of the regions of the polypeptide (anti-EGFRviiiCAR) according to the present disclosure, a nucleotide sequence encoding the target antigen-binding region, a nucleotide sequence encoding the transmembrane region, and a nucleotide sequence encoding the signal transduction region are preferably arranged in this order from a 5' side.

In a case where the signal peptide, the extracellular hinge region, and the like are used, preferably, a nucleotide sequence encoding the signal peptide is arranged on a 5' side of the nucleotide sequence encoding the target antigen-binding region, and a nucleotide sequence encoding the extracellular hinge region is arranged between the nucleotide sequence encoding the target antigen-binding region and the nucleotide sequence encoding the transmembrane region.

The nucleotide sequences encoding these regions may be directly linked to each other, or may be linked to each other via a nucleotide sequence encoding a spacer region.

The spacer region is as described above in the above section of [Chimeric antigen receptor (anti-EGFRviiiCAR)].

In a case where the polypeptide is an anti-EGFRviiiCAR, specific examples of a nucleotide sequence encoding the anti-EGFRviiiCAR include the nucleotide sequence set forth in SEQ ID NO: 11.

The polynucleotide according to the present disclosure is obtained by linking polynucleotides each including a nucleotide sequence encoding each of the regions of the polypeptide (preferably anti-EGFRviiiCAR) to each other directly or via a spacer sequence. The polynucleotide encoding each of the regions of the polypeptide may be obtained by chemical synthesis by a known method based on a nucleotide sequence of each of the regions.

Alternatively, the polynucleotide encoding each of the regions of the polypeptide may be obtained by using DNA extracted from a T cell or the like and cDNA obtained by reverse transcription of RNA extracted from a T cell or the like as a template, and amplifying a polynucleotide encoding each of the regions, for example, by an isothermal amplification method such as a PCR method, a LAMP method, or a TRC method.

The polynucleotide encoding each of the regions thus obtained may be modified by substitution, deletion, addition, or insertion within a range in which the function of each of the regions after translation is not lost.

In addition to the nucleotide sequence encoding an anti-EGFRviiiCAR, the polynucleotide according to the present disclosure may include a control sequence such as a promoter, an enhancer, a poly A addition signal, or a terminator, a nucleotide sequence encoding another protein, and the like.

Examples of the other protein include IL-7 and CCL19. Nucleotide sequences encoding these proteins are available from a known sequence database such as GenBank.

For example, in a case where human IL-7 is used, examples of a nucleotide sequence encoding human IL-7 include an amino acid sequence registered as GenBank number: NM_002190.2 (SEQ ID NO: 49). In a case where human CCL19 is used, examples of a nucleotide sequence encoding human CCL19 include an amino acid sequence registered as GenBank number: NM_006274.2 (SEQ ID NO: 50).

In addition, the nucleotide sequences encoding these proteins are not limited to known ones, and may be any sequences as long as they are nucleotide sequences encoding these proteins, and may be any of a plurality of nucleotide sequences generated by degeneracy of a genetic code. The nucleotide sequences encoding these proteins are preferably codon-optimized according to the species of a cell to be introduced, and are preferably optimized to a human codon in a case of introduction into a human cell.

In addition, the nucleotide sequences encoding these proteins may encode variants of natural IL-7 and natural CCL19. These variants are as described above in the above section of [Cell expressing anti-EGFRviiiCAR (anti-EGFRviiiCAR-expressing cell)].

In addition, examples of the other protein include a suicide gene. The suicide gene is as described in the above section of [Cell expressing anti-EGFRviiiCAR (anti-EGFRviiiCAR-expressing cell)].

A nucleotide sequence encoding the suicide gene is available from a known sequence database such as GenBank. In addition, a sequence such as a vector containing a commercially available suicide gene can also be used.

In a case where the polynucleotide according to the present disclosure includes a nucleotide sequence encoding another protein, a nucleotide sequence encoding a self-cleaving peptide such as a 2A peptide, an internal ribozyme entry site (IRES) sequence, or the like may be interposed between the nucleotide sequence encoding the anti-EGFRviiiCAR and the nucleotide sequence encoding the other protein.

In addition, in a case where there are two or more other proteins, a self-cleaving peptide, IRES, or the like may be interposed between the other proteins. By interposing these sequences, a plurality of proteins can be independently expressed from one promoter.

Examples of the 2A peptide include 2A peptides such as picornavirus, rotavirus, insect virus, aphthovirus, and trypanosoma virus.

As a specific example, an amino acid sequence of the 2A peptide (F2A) of picornavirus is represented by SEQ ID NO: 40. A nucleotide sequence encoding the 2A peptide is preferably codon-optimized according to the species of a cell to be introduced, and is preferably optimized to a human codon in a case of introduction into a human cell. An amino acid sequence at a cleavage position of the 2A peptide is represented by SEQ ID NO: 39, and a protein is translated in a form of being cleaved between proline at a C-terminus and glycine adjacent to proline toward an N-terminus side.

In addition, the polynucleotide according to the present disclosure may have a control sequence such as a promoter, an enhancer, a poly A addition signal, or a terminator for each of protein coding sequences of the nucleotide sequence encoding the polypeptide (preferably anti-EGFRviiiCAR) and the nucleotide sequence encoding another protein. In addition, some protein coding sequences may each independently have a control sequence, and other protein coding sequences may be linked thereto via the 2A peptide, IRES, or the like to have a common control sequence.

### (Vector)

The vector according to the present disclosure preferably includes a nucleotide sequence encoding the polypeptide. The polynucleotide may be in a form of a vector.

The type of vector is not particularly limited, and examples thereof include a generally used expression vector.

The vector may be linear or circular, may be a non-virus vector such as a plasmid, may be a virus vector, or may be a vector based on a transposon.

Examples of the vector include a virus vector, a plasmid vector, an episomal vector, and an artificial chromosome vector.

Examples of the virus vector include a Sendai virus vector, a retrovirus (including lentivirus) vector, an adenovirus vector, an adeno-associated virus vector, a herpesvirus vector, a vaccinia virus vector, a poxvirus vector, a poliovirus vector, a sindbis virus vector, a rhabdovirus vector, a paramyxovirus vector, and an orthomyxovirus vector.

Examples of the plasmid vector include plasmid vectors for animal cell expression, such as pA1-11, pXT1, pRc/CMV, pRc/RSV, and pcDNAI/Neo.

The episomal vector is a vector capable of autonomously replicating extrachromosomally. Examples of the episomal vector include a vector including a sequence necessary for autonomous replication derived from an EV virus (EBV), Simian virus 40 (SV40), or the like as a vector element. Specific examples of the vector element required for autonomous replication include a replication initiation point and a gene encoding a protein that binds to the replication initiation point and controls replication.

Examples thereof include a replication initiation point oriP and an EBNA-1 gene in EBV, and a replication initiation point ori and an SV40LT gene in SV40.

Examples of the artificial chromosome vector include a yeast artificial chromosome (YAC) vector, a bacterial artificial chromosome (BAC) vector, and a P1-derived artificial chromosome (PAC) vector.

Suitable examples of the vector according to the present disclosure include a virus vector, and more suitable examples thereof include a retrovirus vector.

Examples of the retrovirus vector include a pMSGV1 vector (Tamada K et al., Clin Cancer Res 18: 6436-6445 (2012)) and a pMSCV vector (manufactured by Takara Bio Inc.). By using the retrovirus vector, a gene in the vector is incorporated into a genome of a host cell, and can be stably expressed in the host cell for a long period of time.

The vector according to the present disclosure may include a nucleotide sequence encoding a replication initiation point and a protein that binds to the replication initiation point and controls replication, a nucleotide sequence encoding a marker gene such as a drug-resistant gene or a reporter gene, and the like in addition to the nucleotide sequence described in the above section [Polynucleotide including a nucleotide sequence encoding an anti-EGFRviiiCAR] .

The nucleotide sequence encoding the polypeptide according to the present disclosure (preferably an anti-EGFRviiiCAR) is preferably arranged in a vector so as to be expressed under control of an appropriate promoter. In a case where the vector includes a nucleotide sequence encoding another protein, the nucleotide sequences is also preferably arranged in the vector so as to be expressed under control of an appropriate promoter.

Examples of the promoter include an SRα promoter, an SV40 early promoter, a retroviral LTR, a cytomegalovirus (CMV) promoter, a Rous sarcoma virus (RSV) promoter, a herpes simplex virus thymidine kinase (HSV-TK) promoter, an EF1α promoter, a metallothionein promoter, and a heat shock promoter.

In addition, an enhancer of an IE gene of human CMV may be used together with the promoter. Examples thereof include a CAG promoter (containing a cytomegalovirus enhancer, a chicken β-actin promoter, and a poly-A signal site of a β-globin gene). In addition, as described in the above [Polynucleotide including a nucleotide sequence encoding an anti-EGFRviiiCAR], a nucleotide sequence encoding a self-cleaving peptide or IRES may be arranged between protein coding sequences, and transcription may be performed under control of a common promoter.

The vector according to the present disclosure preferably further includes at least one of a nucleotide sequence encoding IL-7 and a nucleotide sequence encoding CCL19 in addition to the nucleotide sequence encoding the polypeptide (preferably an anti-EGFRviiiCAR), and more preferably further includes a nucleotide sequence encoding IL-7 and a nucleotide sequence encoding CCL19 in addition to the nucleotide sequence encoding the anti-EGFRviiiCAR.

The vector preferably includes a nucleotide sequence encoding an anti-EGFRviiiCAR functionally linked to an appropriate promoter, and more preferably, the nucleotide sequence encoding the polypeptide (preferably an anti-EGFRviiiCAR), the nucleotide sequence encoding IL-7, and the nucleotide sequence encoding CCL19 each include a nucleotide sequence encoding a self-cleaving peptide or a nucleotide sequence linked via IRES, and the nucleotide sequence is functionally linked to an appropriate promoter.

### <<Combination of nucleotide sequences included in vector>>

A combination of nucleotide sequences included in the vector is not particularly limited, and a nucleotide sequence encoding the polypeptide (preferably, an anti-EGFRviiiCAR), a nucleotide sequence encoding IL-7, and a nucleotide sequence encoding CCL19 may be carried on separate vectors and introduced into a cell. Two or more nucleotide sequences selected from these nucleotide sequences may be combined, carried on the same vector, and introduced into a cell.

For example, in a case where the polypeptide according to the present disclosure, IL-7, and CCL19 are expressed, nucleotide sequences encoding these may be carried on separate vectors and introduced into a cell, two types of vectors, that is, a vector carrying a nucleotide sequence encoding the polypeptide according to the present disclosure and a nucleotide sequence encoding IL-7 and a vector carrying a nucleotide sequence encoding CCL-19 may be introduced into a cell, two types of vectors, that is, a vector carrying a nucleotide sequence encoding the polypeptide according to the present disclosure and a nucleotide sequence encoding CCL19 and a vector carrying a nucleotide sequence encoding IL-7 may be introduced into a cell, two types of vectors, that is, a vector carrying a nucleotide sequence encoding the polypeptide according to the present disclosure and a nucleotide sequence encoding IL-7 and a vector carrying a nucleotide sequence encoding the polypeptide according to the present disclosure and a nucleotide sequence encoding CCL19 may be introduced into a cell, or one type of vector, that is a vector carrying a nucleotide sequence encoding the polypeptide according to the present disclosure, a nucleotide sequence encoding IL-7, and a nucleotide sequence encoding CCL19 may be introduced into a cell.

Note that, in the present specification, "functionally linked to a promoter" means being linked downstream of the promoter so as to be expressed under control of the promoter. In the above example, the arrangement order of the nucleotide sequence encoding the polypeptide according to the present disclosure, the nucleotide sequence encoding IL-7, and the nucleotide sequence encoding CCL19 is not particularly limited, and may be any arrangement order.

### (Method for producing cell expressing polypeptide)

A method for producing a cell expressing a polypeptide according to the present disclosure (hereinafter, also simply referred to as "method for producing a cell") preferably includes introducing the polynucleotide into a cell. In addition, the method for producing a cell expressing a polypeptide according to the present disclosure preferably includes introducing the polynucleotide or vector into a cell.

A method for introducing the polynucleotide or vector into a cell is not particularly limited, and a known method can be used. Examples thereof include a virus infection method, a lipofection method, a microinjection method, a calcium phosphate method, a DEAE-dextran method, an electroporation method, a method using a transposon, and a particle gun method.

In a case where the vector used in the method for producing a cell is a retrovirus vector, an appropriate packaging cell may be selected based on an LTR sequence and a packaging signal sequence included in the vector, and a retroviral particle may be prepared using the selected packaging cell.

Examples of the packaging cell include PG13, PA317, GP+E-86, GP+envAm-12, and Psi-Crip. In addition, a 293 cell or a 293T cell having a high transfection efficiency can also be used as the packaging cell.

Since various retrovirus vectors and packaging cells that can be used for packaging the vectors are widely commercially available, these commercially available products may be used. For example, a GP2-293 cell (manufactured by Takara Bio Inc.), a Plat-GP cell (manufactured by Cosmo Bio Inc.), a PG13 cell (ATCCCRL-10686), and a PA317 cell (ATCCCRL-9078) can be used, and a commercially available kit such as Retrovirus packaging Kit Eco (manufactured by Takara Bio Inc.) may be used.

In a case where another exogenous protein such as IL-7, CCL19, or a suicide gene is expressed in a cell expressing the polypeptide according to the present disclosure, a nucleotide sequence encoding each of these other proteins may be included in a vector including a nucleotide sequence encoding the polypeptide according to the present disclosure (preferably, an anti-EGFRviiiCAR), or may be included in another vector. In a case where a nucleotide sequence encoding another protein is included in another vector, the vector can be introduced into a cell simultaneously with or separately from a vector including a nucleotide sequence encoding the polypeptide according to the present disclosure (preferably an anti-EGFRviiiCAR).

In addition, the cell expressing the polypeptide according to the present disclosure may be produced by incorporating a polynucleotide including a nucleotide sequence encoding the polypeptide according to the present disclosure into a genome of the cell so as to be able to be expressed under control of an appropriate promoter using a known gene editing technique or the like.

Examples of the gene editing technique include a technique using an endonuclease such as zinc finger nuclease, transcription activation-like effector nuclease (TALEN), clustered regularly interspaced short palindromic repeat (CRISPR)-Cas system, or pentatricopeptide repeat (PPR).

Similarly, in a case where another exogenous protein is expressed in a cell expressing the polypeptide according to the present disclosure, a polynucleotide including a nucleotide sequence encoding another exogenous protein may be incorporated into a genome of the cell so as to be able to be expressed under control of an appropriate promoter using a gene editing technique or the like. Examples thereof include: a method for incorporating a polynucleotide including a nucleotide sequence encoding the polypeptide according to the present disclosure (or another protein) functionally linked to an appropriate promoter into a non-coding region or the like of a cell genome.; and a method for incorporating a polynucleotide including a nucleotide sequence encoding the polypeptide according to the present disclosure (or another protein) downstream of an endogenous promoter of a cell genome.

Examples of the endogenous promoter include promoters of TCRα and TCRβ.

### -Method for confirming expression-

After a polynucleotide or a vector including a nucleotide sequence encoding the polypeptide according to the present disclosure is introduced into a cell, expression of the polypeptide in the cell can be confirmed by a known method such as flow cytometry, RT-PCR, Northern blotting, Western blotting, ELISA, or fluorescent immunostaining. In addition, expression of another exogenous protein such as IL-7 or CCL19 can be similarly confirmed by a known method.

### (Pharmaceutical composition)

A pharmaceutical composition according to the present disclosure preferably contains the above cell.

A cell expressing the polypeptide according to the present disclosure exhibits specific cytotoxic activity against a cell expressing EGFRviii. Therefore, the cell expressing the polypeptide can be used for treating or preventing a disease in which the cell expressing the polypeptide is involved. Since EGFRviii is expressed in a wide range of tumor cells including lung cancer, neuroblastoma, glioma, melanoma, malignant mesothelioma, myeloma, and the like, a pharmaceutical composition containing a cell expressing the polypeptide can be used as a pharmaceutical composition for treating or preventing a tumor.

The tumor may be generated in any of a bone tissue, a cartilage tissue, an adipose tissue, a muscle tissue, a vascular tissue, and a hematopoietic tissue. Examples of the tumor include: cancers such as glioma, melanoma, malignant mesothelioma, lung cancer, pancreatic cancer, head and neck cancer, liver cancer, uterine cancer, bladder cancer, biliary tract cancer, esophageal cancer, testicular tumor, thyroid cancer, brain tumor, prostate cancer, large bowel cancer, renal cancer, ovarian cancer, breast cancer, adenocarcinoma, squamous cell cancer, adenosquamous cancer, anaplastic cancer, large cell cancer, small cell cancer, skin cancer, vaginal cancer, neck cancer, spleen cancer, tracheal cancer, bronchial cancer, small intestine cancer, stomach cancer, gallbladder cancer, and testicular cancer; sarcomas such as osteosarcoma, chondrosarcoma, Ewing's sarcoma, malignant hemangioendothelioma, malignant schwannoma, and soft tissue sarcoma; blastomas such as neuroblastoma, hepatoblastoma, medulloblastoma, nephroblastoma, pancreatic blastoma, pleuropulmonary blastoma, and retinoblastoma; a germ cell tumor; and hematologic cancers such as lymphoma, leukemia, and myeloma, but are not limited thereto.

In particular, the pharmaceutical composition according to the present disclosure is suitable as a pharmaceutical composition for treating or preventing a tumor expressing EGFRviii.

Whether or not a tumor is expressing EGFRviii can be confirmed, for example, by a known method using an anti-EGFRviii antibody or the like.

Examples of the known method for confirming expression of EGFRviii include flow cytometry, ELISA, immunostaining, and fluorescent immunostaining.

A cell expressing either or both of IL-7 and CCL19 (preferably expressed from an exogenous nucleic acid) in addition to the polypeptide according to the present disclosure can exhibit strong cytotoxic activity even against a solid tumor as long as the solid tumor is a tumor expressing EGFRviii. Therefore, a pharmaceutical composition containing the cell expressing the polypeptide according to the present disclosure and either or both of IL-7 and CCL19 can be particularly suitably used for a solid tumor expressing EGFRviii. Therefore, the pharmaceutical composition for treating or preventing a solid tumor, the pharmaceutical composition containing the cell expressing the polypeptide according to the present disclosure and either or both of IL-7 and CCL19, is a suitable example of the pharmaceutical composition according to the present disclosure.

The "solid tumor" means a tumor other than hematologic cancer generated from a hematopoietic tissue, and includes epithelial cell cancer and non-epithelial cell cancer.

The pharmaceutical composition according to the present disclosure may contain, in addition to the cell expressing the polypeptide according to the present disclosure, another component such as a pharmaceutically acceptable carrier.

Examples of the other component include a pharmaceutically acceptable carrier, a T cell activation factor such as cytokine, an immunostimulant, an immune checkpoint inhibitor, a cell expressing another CAR, and an anti-inflammatory agent, but are not limited thereto. Examples of the pharmaceutically acceptable carrier include a cell culture medium, physiological saline, a phosphate buffer, and citrate buffer.

The pharmaceutical composition according to the present disclosure can be administered by a known method, but preferably can be administered to a patient by injection or infusion. An administration route is preferably intravenous administration, but is not limited thereto, and administration may be performed, for example, by injection into a tumor.

The pharmaceutical composition according to the present disclosure can contain a therapeutically effective amount of the cell expressing the polypeptide according to the present disclosure.

In the present specification, the "therapeutically effective amount" means an amount of a drug effective for treating or preventing a disease. The therapeutically effective amount may vary depending on a disease condition, an age, a sex, a weight, and the like of an administration target. In the pharmaceutical composition according to the present disclosure, the therapeutically effective amount of the cell expressing the polypeptide is, for example, an amount in which the cell expressing the polypeptide can suppress proliferation of a tumor, and is preferably an amount that leads to reduction of a tumor.

An administration amount and an administration interval of the pharmaceutical composition according to the present disclosure can be appropriately selected according to an age, a sex, a weight, and the like of an administration target, a type, a progression degree, a symptom, and the like of a disease, an administration method, and the like.

As the administration amount, a therapeutically effective amount can be administered, and for example, in one administration, the number of administered cells is 1 × 10⁴ to 1 × 10¹⁰, preferably 1 × 10⁵ to 1 × 10⁹, and more preferably 5 × 10⁶ to 5 × 10⁸.

The administration interval of the pharmaceutical composition according to the present disclosure can be, for example, every week, every 10 days to 30 days, every month, every three months to six months, or every year. In addition, since the cell expressing the polypeptide according to the present disclosure can autonomously proliferate in a body of an administration target, administration can be performed only once. In addition, the number of cells expressing the polypeptide in the body may be monitored after administration, and a time of administration may be determined according to a result thereof.

In addition, the pharmaceutical composition according to the present disclosure can be used in combination with another anticancer agent. Examples of the other anticancer agent include an alkylating agent such as cyclophosphamide, an antimetabolite such as pentostatin, a molecular targeting agent such as rituximab, a kinase inhibitor such as imatinib, a proteasome inhibitor such as bortezomib, a calcineurin inhibitor such as cyclosporine, an anticancer antibiotic such as idarubicin, a plant alkaloid such as irinotecan, a platinum preparation such as cisplatin, a hormone therapy agent such as tamoxifen, and an immunomodulatory agent such as nivolumab or pembrolizumab, but are not limited thereto.

In addition, the administration target of the pharmaceutical composition according to the present disclosure is not particularly limited, and may be a human, a monkey, a dog, or the like.

In another aspect, the present disclosure provides: 1) use of a cell expressing the polypeptide according to the present disclosure in manufacture of a pharmaceutical composition for treating or preventing a tumor; 2) a method for treating or preventing a tumor expressing EGFRviii, including administering a cell expressing the polypeptide according to the present disclosure to a target (for example, a patient suffering from a tumor expressing EGFRviii or a patient with surgical resection of a tumor); 3) a cell expressing the polypeptide according to the present disclosure to be used for treating or preventing a tumor; and 4) use of a cell expressing the polypeptide according to the present disclosure for treating or preventing a tumor.

Furthermore, in another aspect, the present disclosure also provides a kit for producing a cell expressing the polypeptide according to the present disclosure, including the vector according to the present disclosure. The kit is not particularly limited as long as it includes the vector according to the present disclosure, and may include an instruction for producing a cell expressing the polypeptide according to the present disclosure, a reagent used for introducing the vector into a cell, and the like.

### Examples

Hereinafter, the present disclosure will be described with reference to Examples, but the present disclosure is not limited to the following Examples.

### (Example 1) Production of anti-EGFRviii antibody

According to a usual method, mice were immunized with a peptide derived from EGFRviii, and screening was performed using HEK EGFRviii cells expressing EGFRviii and HEK293 cells not expressing EGFRviii to obtain 48 hybridomas producing antibodies that bind to EGFRviii.

Furthermore, screening by FACS analysis was performed using CLTH/EGFRviii cells expressing EGFRviii (manufactured by Celther Polska) and 293 cells not expressing EGFRviii (manufactured by National Research and Development Agency, Pharmaceutical infrastructure, Health and Nutrition Research, JCRB Cell Bank), and three clones of 1B10, 2H9, and 3C2 were selected.

Single cloning was performed on these clones. Using strong binding to an EGFRviii-expressing strain, weak binding to an EGFRviii-non-expressing strain, and fast cell proliferation as criteria for selecting a clone, a 1B10-1A12 clone (SEQ ID NOs: 1 and 2), a 2H9-1B5 clone (SEQ ID NOs: 51 and 52), and a 3C2-1B10 clone (SEQ ID NOs: 53 and 54) were selected and used for subsequent experiments.

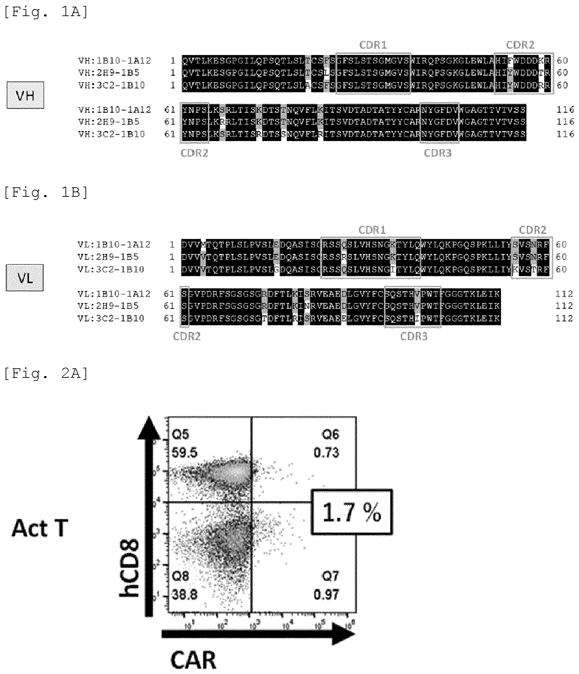

Results of analyzing amino acid sequences of the three clones and performing alignment are illustrated in Figs. 1A and 1B.

As illustrated in Figs. 1A and 1B, the three clones were found to exhibit high sequence identity to each other. CDR illustrated in Figs. 1A and 1B is defined by Kabat.

### (Example 2) Study of mouse anti-EGFRviiiCAR-T cell

### <Design of anti-EGFRviiiscFv based on 1B10-1A12 clone>

First, using VH and VL of the 1B10-1A12 clone and linkers 15 and 25, eight types of anti-EGFRviiiscFvs presented in Table 1 below were designed. Note that since SEQ ID NOs: 13 and 15 in this Table are sequences after conversion to (SG) described below, Ser at position 164 of SEQ ID NO: 14 and Ser at position 174 of SEQ ID NO: 16 are actually replaced with Asn.

**[Table 1]**

| | Structure | SEQ ID NO |
|---|---|---|
| S#020 | VL15VH (NG) | 13 |
| S#021 | VH15VL (NG) | 14 |
| S#022 | VL25VH (NG) | 15 |
| S#023 | VH25VL (NG) | 16 |
| S#024 | VL15VH (SG) | 3, 41, 1 |
| S#025 | VH15VL (SG) | 1, 41, 3 |
| S#026 | VL25VH (SG) | 3, 42, 1 |
| S#027 | VH25VL (SG) | 1, 42, 3 |

In Table 1, VL15VH means that a VH region of the 1B10-1A12 clone, the linker 15, and a VL region of the 1B10-1A12 clone are included sequentially from an N-terminus, (NG) means that CDR1 of VL contains an NG (Asn-Gly) sequence, and (SG) indicates that the NG sequence is converted into an SG (Ser-Gly) sequence.

In the NG (Asn-Gly) sequence in CDR1 of VL, deamidation is likely to occur, and the deamidation may reduce antigen binding ability. Therefore, a sequence obtained by changing the NG (Asn-Gly) sequence to the SG (Ser-Gly) sequence was produced.

### <Synthesis of scFv sequence and DNA fragment of anti-EGFRviiiCAR>

DNA fragments encoding the amino acid sequences of the eight types of anti-EGFRviiiscFvs were synthesized.

### <Production of anti-EGFRviiiCAR-expressing vector>

### [Production of anti-EGFRviiiIL-7/CCL19CAR-expressing vector expressing IL-7/CCL19 and HSV-TK]

For the eight types of anti-EGFRviiiscFvs, first, a third generation CAR construct including an anti-EGFRviiiscFv, a human CD8 transmembrane region, and a human CD28-4-1BB-CD3ζ intracellular signal transduction region sequentially from an N-terminus side was produced according to the method described in WO 2017/159736 A.

F2A, which is a 2A peptide (SEQ ID NO: 39), was added to a C-terminus of the construct, and human IL-7-F2A-human CCL19-F2A-HSV-TK was further added downstream thereof. Amino acid sequences are illustrated in Table 2 below.

Next, each of EGFRviiiscFvDNA fragments synthesized by the method described in the above section "Synthesis of scFv sequence and DNA fragment of anti-EGFRviiiCAR" was inserted into the anti-EGFRviiiscFv region in the pMSGV vector by restriction enzyme (NcoI and NotI) treatment and ligation to produce each "IL-7/CCL19-expressing-anti-EGFRviiiCAR vector".

**[Table 2]**

| SEQ ID NO | Sequence name | Amino acid sequence |
|---|---|---|
| 20,22 | hCD8 | FVPVFLPAKPTTTPAPRPPTPAPTIASQPLSLRPEACRPAAGGAVHTRGIDFACDIYIWAPLAGTCGVLLLSLVILYCNHRN |
| 26 | CD28_A_aa | RSKRSRLLHSDYMNMTPRRPGPIRKHYQPYAPPRDFAAYRS |
| 27 | 4-1 BB_A_aa | RFSVVKRGRKKLLYIFKQPFMRPVQTTQEEDGCSCRFPEEEEGGCE |
| 25 | CD3z_A_aa | LRVKFSRSADAPAYQQGQNQLYNELNLGRREEYDVLDKRRGRDPEMGGKPRRKNPQEGLYNELQKDKMAEAYSEIGMKGERRRG KGHDGLYQGLSTATKDTYDALHMQALPPR |
| 28 | IL-7 human_aa | MFHVSFRYIFGLPPLILVLLPVASSDCDIEGKDGKQYESVLMVSIDQLLDSMKEIGSNCLNNEFNFFKRHICDANKEGMFLFRAARK LRQFLKMNSTGDFDLHLLKVSEGTTILLNCTGQVKGRKPAALGEAQPTKSLEENKSLKEQKKLNDLCFLKRLLQEIKTCWNKILMG TKEH |
| 29 | CCL19 human_aa | MALLLALSLLVLWTSPAPTLSGTNDAEDCCLSVTQKPIPGYIVRNFHYLLIKDGCRVPAVVFTTLRGRQLCAPPDQPWVERIIQRLQ RTSAKMKRRSS |
| 39 | 2A peptide cleavage region | DXEXNPGP |
| 40 | F2A_aa | GSGVKQTLNF DLLKLAGDVE SNPGP |
| 41 | linker 15_aa | GGGGSGGGGSGGGGS |
| 42 | linker 25_aa | SSADDAKKOAAKKDDAKKDDAKKDG |

### <Production of retrovirus into which IL-7/CCL19-expressing-anti-human EGFRviiiCAR vector is introduced>

A retrovirus for introducing a gene into a T cell was produced.

First, using a transfection reagent (product name: Lipofectamine 3000 (manufactured by Life Technologies)), each of the above IL-7/CCL19-expressing-anti-EGFRviiiCAR vectors and p-Ampho plasmid (manufactured by Takara Bio Inc.) were transfected into a GP2-293 packaging cell line (manufactured by Takara Bio Inc.) to produce a retrovirus into which the IL-7/CCL19-expressing-anti-EGFRviiiCAR vector was introduced.

Supernatants containing retroviruses into which the anti-EGFRviii vectors were introduced, respectively were collected 48 hours after transfection.

As a culture solution for the GP2-293 cell, DMEM containing 10% FCS and 1% Penicillin-Streptmycin (manufactured by FUJIFILM Wako Pure Chemical Corporation) was used. As a culture solution for a T cell used in Examples described later, CTS Immune CELLSR (manufactured by Gibco), 1% Penicillin-Streptmycin (manufactured by FUJIFILM Wako Pure Chemical Corporation), 2.5 ug/mL amphotericin B (manufactured by Bristol Myers Squibb Company), and OpTmizer containing 2 mM L-glutamine (manufactured by Gibco) was used.

### <Transduction into T cell>

2 × 10⁶ peripheral blood mononuclear cells collected from blood of a healthy donor were cultured together with IL-2 (manufactured by Miltenyi Biotec) on a plate on which an anti-CD3 monoclonal antibody (5 µg/mL) and RetroNectin (registered trademark) (manufactured by Takara Bio Inc., 25 µg/mL) were immobilized for activation of T cells in a 5% CO₂ incubator at 37°C for three days.

On Day 2 after start of the culture, the above-produced supernatant containing the retrovirus into which the IL-7/CCL19-expressing-anti-human EGFRviiiCAR vector was introduced was added in an amount of 500 µL per well to a surface-untreated 24 well plate coated with 25 µg/mL RetroNectin (manufactured by Takara Bio Inc.) in advance, and centrifuged at 2000 g for two hours to produce a retrovirus-preloaded plate.

Two retrovirus-preloaded plates were produced for each retrovirus. After completion of centrifugation, the plates were washed with 1.5% BSA/PBS and stored at 4°C until use.

On Day 3 of culture, the activated cells were collected from the plate, and a cell suspension was prepared such that the number of cells was 1 × 10⁵ cells/mL. The cell suspension was added to the retrovirus-preloaded plate in an amount of 1 mL per well, and cultured in a 5% CO₂ incubator at 37°C for 24 hours in the presence of IL-2 to perform first retroviral infection.

On the next day (Day 4 of culture), the cell solution of each well was transferred to the second stored retrovirus-preloaded plate, centrifuged at 500 g for one minute, and then cultured at 37°C for four hours to perform second infection.

After culture at 37°C for four hours, 1 mL of the cell suspension of each well was transferred to a new 12 well cell culture plate, diluted four times with a new culture solution containing IL-2 (OpTmizer), and cultured in a 37°C in a 5% CO₂ incubator.

The peripheral blood mononuclear cells were cultured until Day 7 counted from start of culture to obtain an anti-EGFRviiiCAR-T cell (hereinafter, also referred to as "PRIME EGFRviii CAR-T cell") which is a T cell into which the IL-7/CCL19-expressing-anti-EGFRviiiCAR vector is introduced.

The anti-PRIME EGFRviii CAR-T cell contains a nucleic acid encoding an exogenous anti-human EGFRviiiCAR, a nucleic acid encoding an exogenous IL-7, and a nucleic acid encoding an exogenous CCL 19.

At the same time, as a CAR negative cell control, a "CAR-IL-7-CCL19 non-expressing-T cell" (a non-gene-introduced cell: non-infection or an activated T cell: ActT) that activated peripheral blood mononuclear cells obtained from the same healthy donor by a similar method but was not retrovirally infected was produced.

### <Confirmation of expression of CAR>

### [Flow cytometry analysis]

An expression level of the PRIME EGFRviii CAR-T cells was measured by flow cytometry.

The activated T cells and the PRIME EGFRviii CAR-T cells were caused to react with a recombinant EGFRviii protein and an allophycocyanin (APC)-labeled anti-CD8 monoclonal antibody (manufactured by Affymetrix, Inc.) to perform staining.

CytoFLEX S (manufactured by Beckman Coulter, Inc.) was used as the flow cytometer, and FlowJo software (manufactured by Tree Star, Inc.) was used for data analysis.

### (Results)

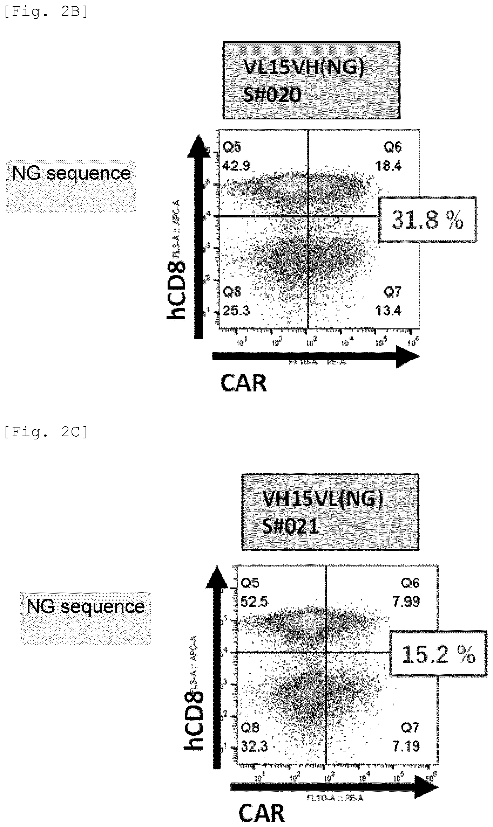

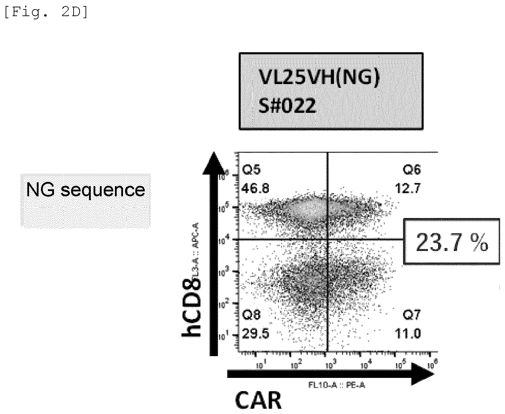

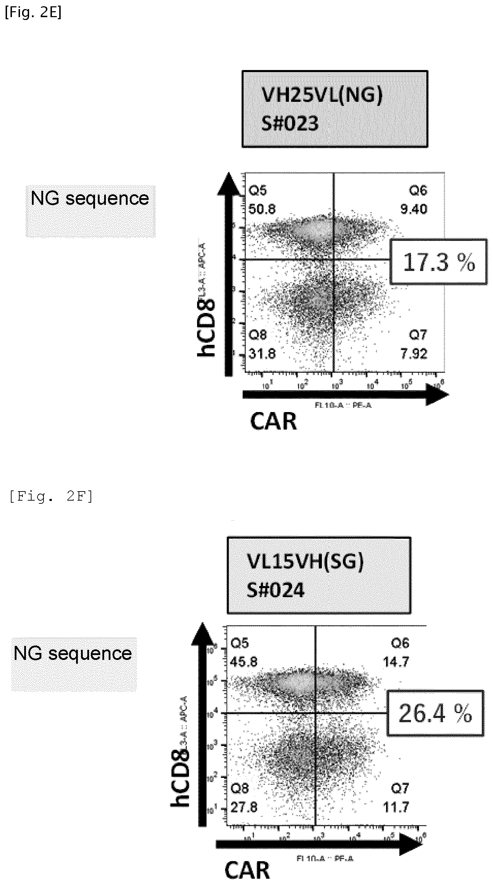

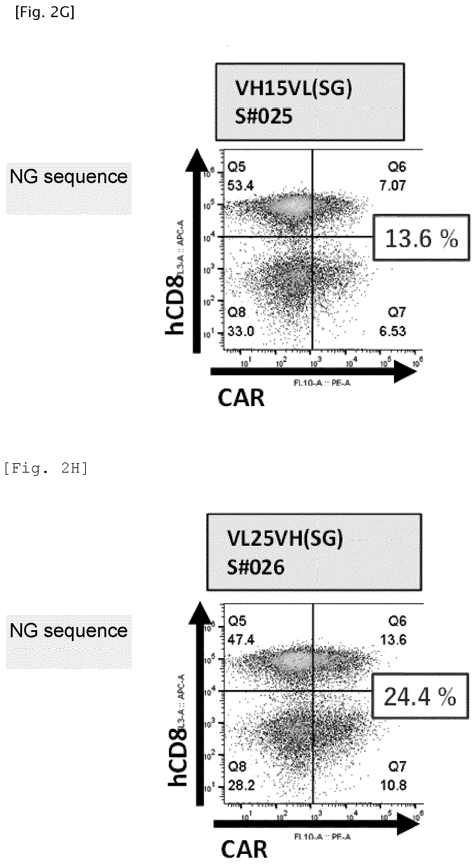

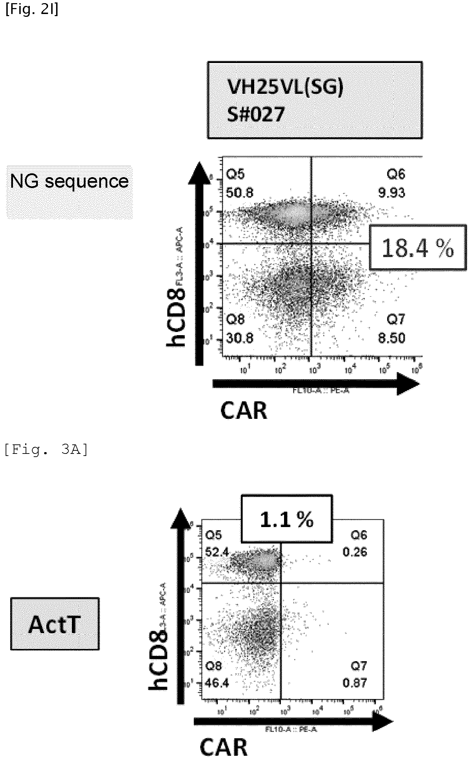

The results are illustrated in Figs. 2A to 2H. In each of Figs. 2A to 2H, the horizontal axis represents expression of CAR, and the vertical axis represents expression of CD8.

As illustrated in Figs. 2A to 2H, expression of CD8 was observed, but expression of CAR was not observed in activated T cells (ActT in Figs. 2A to 2H). Meanwhile, the "PRIME EGFRviii CAR-T cells", which were T cells into which the IL-7/CCL19-expressing-anti-human EGFRviiiCAR vector was introduced, exhibited a sufficiently high expression ratio regardless of the type of scFv. In particular, expression ratios tended to be high in VL15VH and VL25VH each having VL on an N-terminus side tended to be high.

### <Production of anti-EGFRviiiCAR-T cells based on 2H9-1B5 clone and 3C2-1B10 clone>

PRIME EGFRviii CAR-T cells based on a 2H9-1B5 clone and a 3C2-1B10 clone were produced in a manner similar to that based on the 1B10-1A12 clone. For each scFv, a VL25VH (SG) type was used.

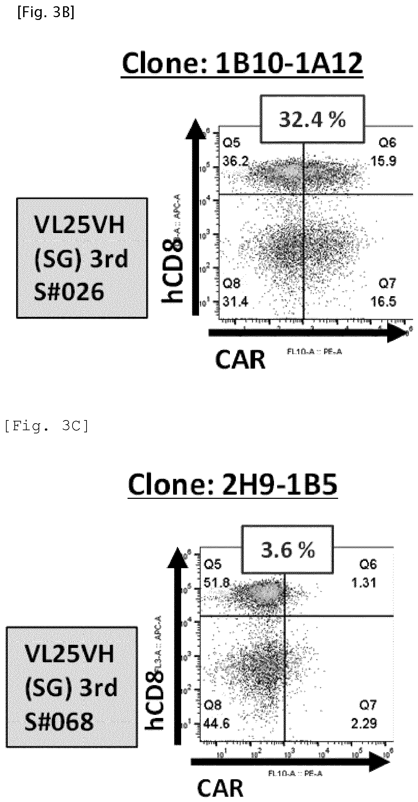

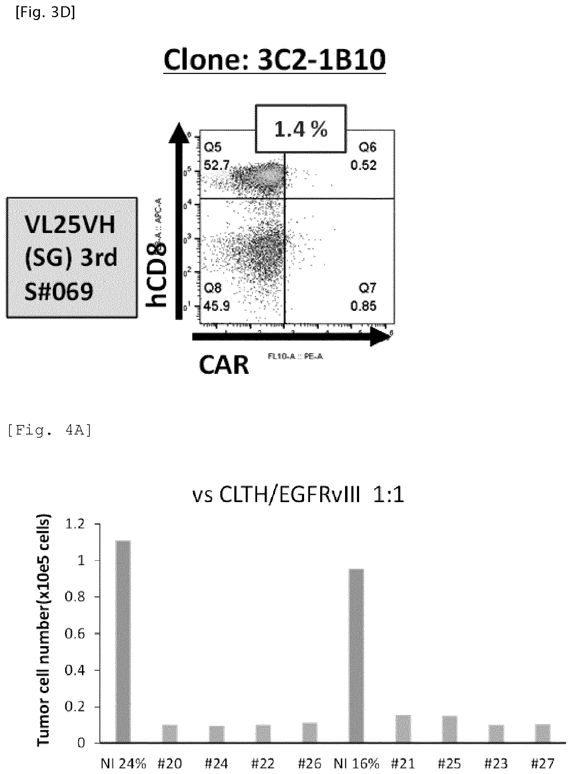

Results of measuring the expression ratio are illustrated in Figs. 3A to 3D.

Despite the very high VH and VL sequence identity with the 1B10-1A12 clone, a flow cytometer measurement result of the PRIME EGFRviii CAR-T cells based on the 1B10-1A12 clone was 32.4%, whereas flow cytometer measurement results of the PRIME EGFRviii CAR-T cells based on the 2H9-1B5 clone and the 3C2-1B10 clone were extremely low values of 3.6% and 1.40, respectively. This is considered to be due to low expression ratios of the 2H9-1B5 clone and the 3C2-1B10 clone and/or low binding affinity thereof to the recombinant EGFRviii protein.

From the above, in the following Examples, the 1B10-1A12 clone was used.

### <Measurement of cytotoxic activity of CAR-T cells>

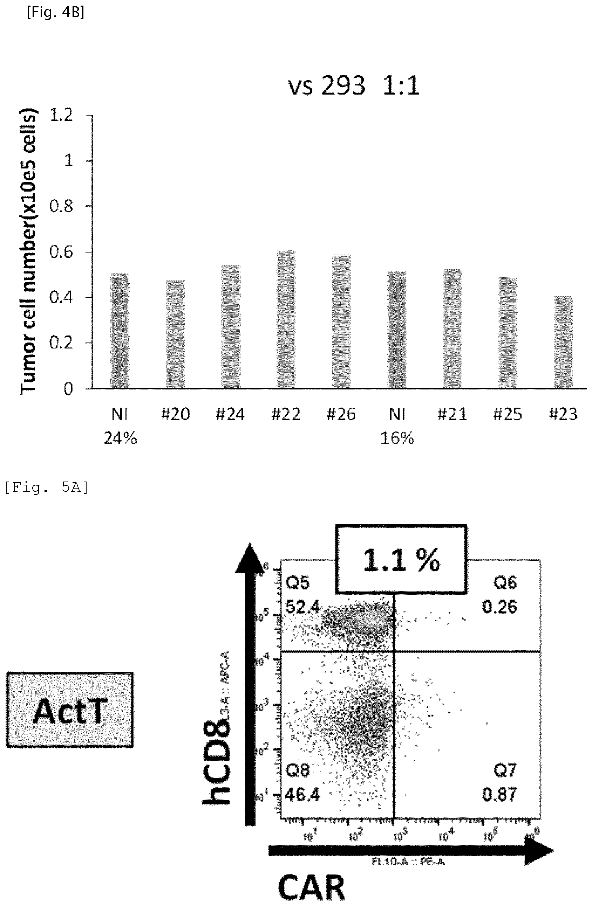

For the PRIME EGFRviii CAR-T cells based on the 1B10-1A12 clone, results of measuring cytotoxic activity of the PRIME EGFRviii CAR-T cells with a ratio (E : T ratio) between CAR-T cells and EGFRviii-expressing cells (CLTH/EGFRviii cells) or EGFRviii-non-expressing cells (293 cells) set to 1 : 1 are illustrated in Figs. 4A and 4B. In Figs. 4A and 4B, NI represents a non-infected cell, and means that a CAR gene is not introduced into the cell. In addition, NI 24% means that the concentration of the cells was adjusted to 24% according to an expression ratio of CAR, and similarly, NI 16% means that the concentration of the cells was adjusted to 160.

As illustrated in Figs. 4A and 4B, in the scFv including the NG sequence and the scFv including the SG sequence, there was no significant difference in expression ratio and cytotoxic activity.

As illustrated in Fig. 4A, the PRIME EGFRviii CAR-T cells exhibited specific cytotoxic activity against EGFRviii-expressing cells regardless of the type of scFv.

From the above results, S#024 VL15VH (SG) and S#026 VL25VH (SG) were used in the following experiments.

### <Study of second generation construct>

For the anti-EGFRviiiCAR having S#024 VL15VH (SG) and S#026 VL25VH (SG) based on the 1B10-1A12 clone as scFvs, use a second generation CAR construct was considered instead of the third generation CAR construct.

The second generation construct includes an anti-EGFRviiiscFv, a human CD8 transmembrane region, 4-1BB, and a CD3ζ intracellular signal transduction region sequentially from an N-terminus side.

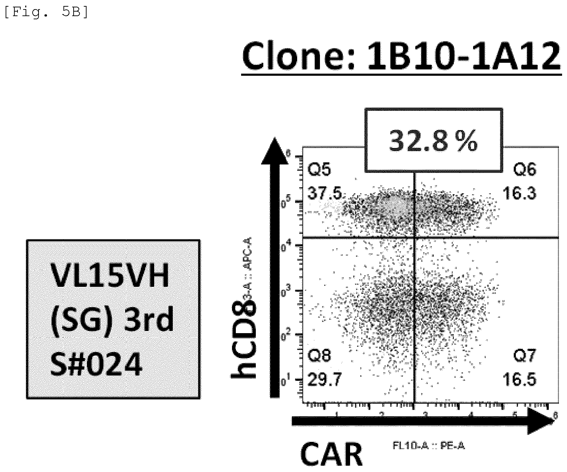

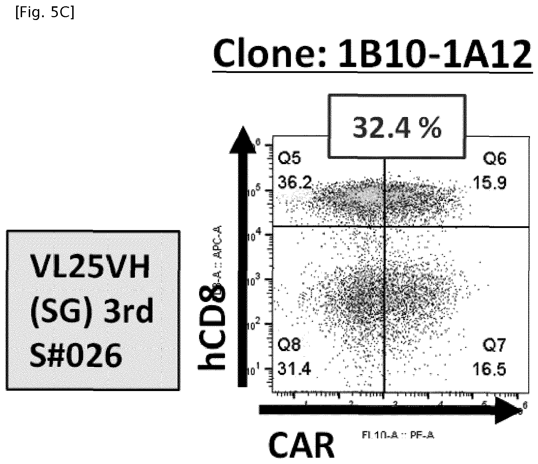

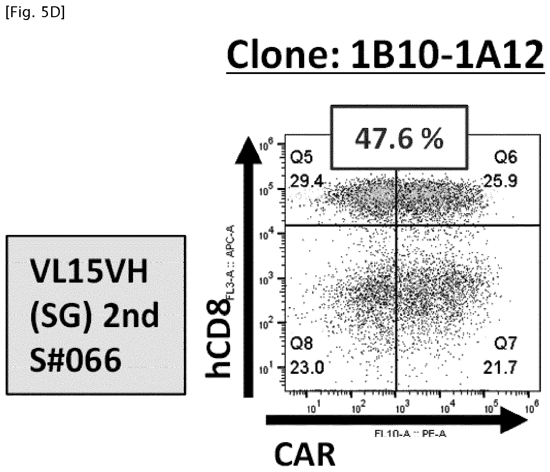

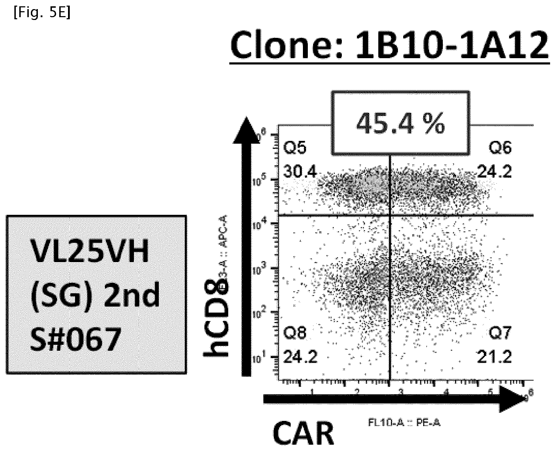

Results of measuring an expression ratio are illustrated in Figs. 5A to 5E.

The second generation construct tended to have a higher expression ratio than the third generation construct.

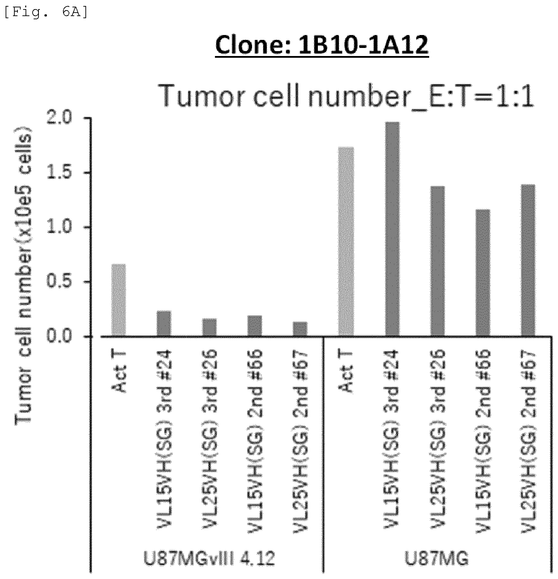

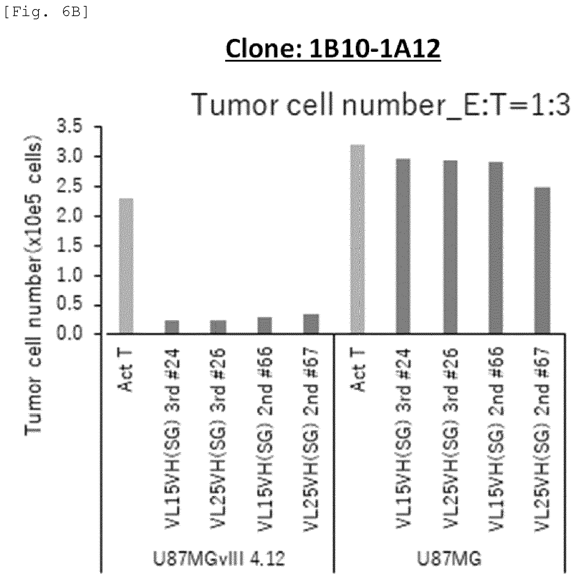

Figs. 6A and 6B illustrate results of measuring cytotoxic activity of the PRIME EGFRviii CAR-T cells by changing the ratio (E : T ratio) between the PRIME EGFRviii CAR-T cells and the EGFRviii-expressing cells (glioblastoma cells U87MGviii 4.12 cells) or the EGFRviii-non-expressing cells (U87MG cells). As illustrated in Figs. 6A and 6B, regardless of scFv configuration and regardless of whether the construct was the second generation or the third generation, all PRIME EGFRviiiCAR-T cells exhibited cytotoxic activity specifically to EGFRviii-expressing cells. Note that Act T in Figs. 6A and 6B means activated T cells, and was used as a control.

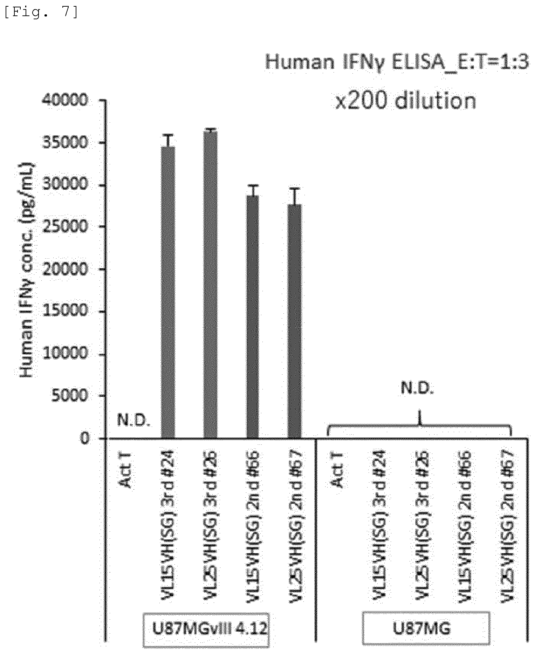

Furthermore, results of measuring an IFNγ concentration of a culture supernatant when the E : T ratio was 1 : 3 is illustrated in Fig. 7. As illustrated in Fig. 7, PRIME EGFRviii CAR-T cells were found to secrete IFNγ only when being co-cultured with EGFRviii-expressing cells. Note that Act T in Fig. 7 means activated T cells, and was used as a control.

### (Example 3) Study of human PRIME EGFRviii CAR-T cells

A scFv was humanized to produce PRIME-EGFRviii CAR-T cells #744 having a VL25VH type scFv using a VH including the amino acid sequence set forth in SEQ ID NO: 4 and a VL including the amino acid sequence set forth in SEQ ID NO: 5. An NG sequence in the scFv was converted into an SG sequence, and the second generation construct was used. The following sequences were used as a signal sequence and a transmembrane region.

### [Signal sequence]

IgG SS: MDWTWRILFLVAAATGAHS (SEQ ID NO: 23)

### [Transmembrane region]

CD8a short:

TTTPAPRPPTPAPTIASQPLSLRPEACRPAAGGAVHTRGLDFACDIYIWAPLAGTCGVL

LLSLVITL (SEQ ID NO: 30)

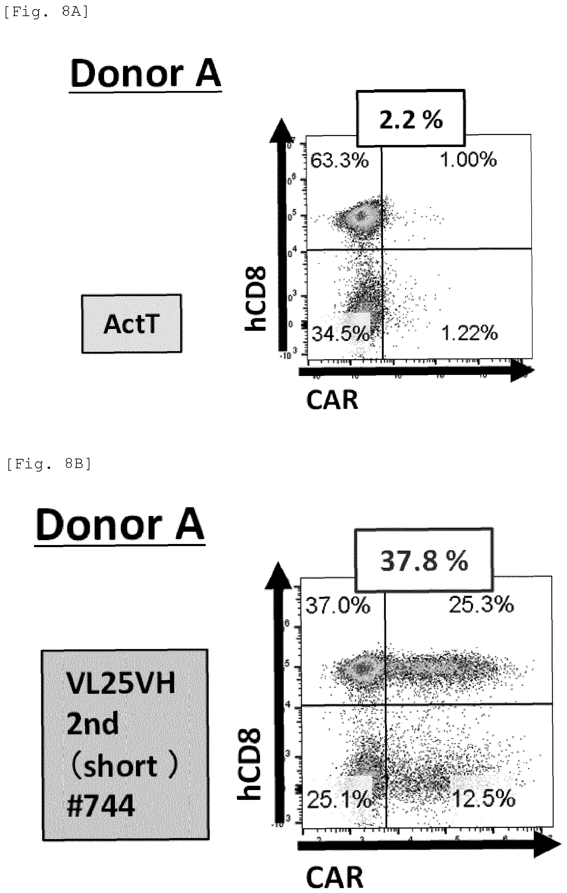

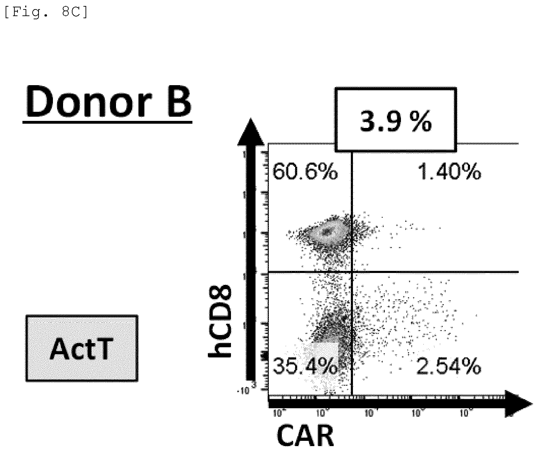

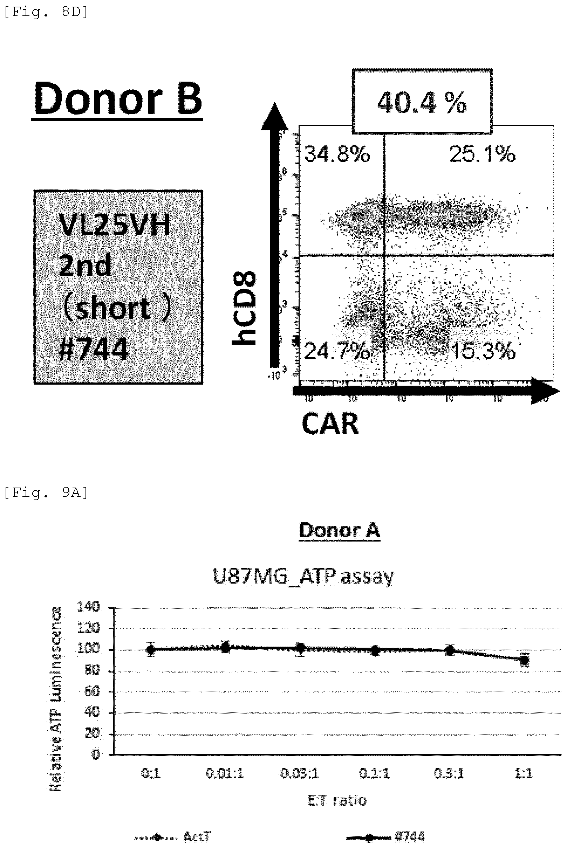

Results of measuring expression ratios of samples obtained from two healthy donors are illustrated in Figs. 8A to 8D. All CARs exhibited high expression ratios.

### <Measurement of cytotoxic activity of EGFRviii CAR-T cells targeting tumor cells by ATP assay>

ATP assays were performed by variously changing a ratio (E : T ratio) of PRIME EGFRviii CAR-T cells to tumor cells, and cytotoxic activity of the PRIME EGFRviii CAR-T cells to tumor cells was measured.

### -Preparation of tumor cell culture medium-

To 500 mL of a MEM medium, 50 mL of FBS and 5 mL of Penicillin-Streptomycin, Liquid were added to prepare a tumor cell culture medium (also referred to as a tumor cell medium).

Tumor cells (glioblastoma cells U87MGviii 4.12 described later) were diluted to 5 × 10⁵ cell/mL in the tumor cell medium prepared above, and the diluted tumor cells were dispensed into a collagen-coated 96 well plate at 100 µL/well.

The cell number and viability of the PRIME EGFRviii CAR-T cells were measured with a cell counter.

CAR positive cells were suspended in the tumor cell medium such that the CAR positive cells had a concentration of 5 × 10⁵ cells/mL from data of the cell counter and a CAR positive ratio to prepare a CAR-T cell liquid.

The CAR-T cell liquid was serially diluted with the tumor cell medium and added to the well plate into which the tumor cells were dispensed at 100 µL/well to perform preparation such that the E : T ratio was 0 : 1, 0.01 : 1, 0.03 : 1, 0.1 : 1, 0.3 : 1, or 1 : 1.

The 96 well plate was put into a CO₂ incubator set at 37°C and 5 vol% CO₂, and co-culture was performed for 48 hours.

The tumor cell culture medium was removed from the well plate taken out of the CO₂ incubator and washed with PBS. Frozen stored CellTiter-Gro Reagent (manufactured by Promega Corporation) was thawed in a light-shielded manner. Thereafter, the whole amount of one vial of CellTiter-Gro substrate (manufactured by Promega Corporation) was put into 10 mL of CellTiter-Gro buffer (manufactured by Promega Corporation), mixed, and dispensed into a 96 well plate at 100 µL/well.

A lid of the 96 well plate was covered with aluminum foil, and the 96 well plate was shaken at 25°C and 200 revolutions per minute (rpm) for 10 minutes to cause a reaction with ATP. Immediately after the reaction, luminescence was measured (Shake 5 sec (60 rpm), Delay 30 se) with a plate reader, and the number of living cells was measured.

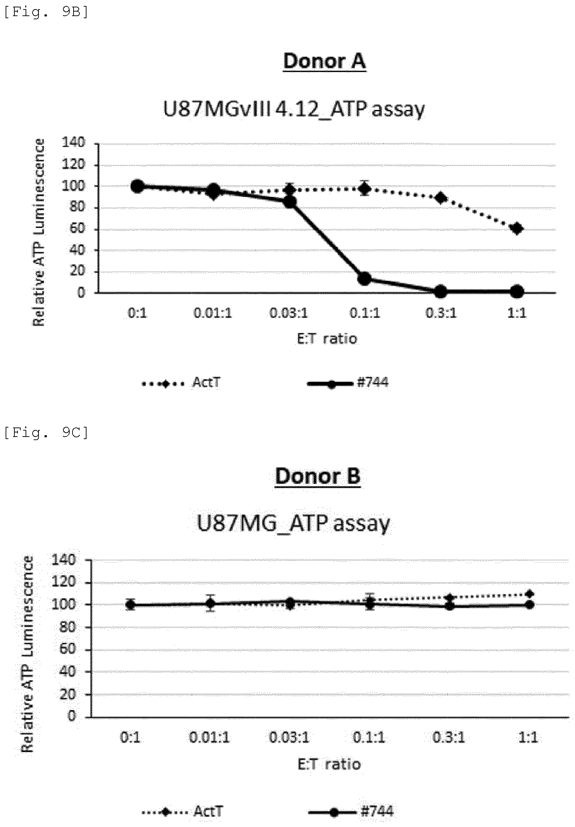

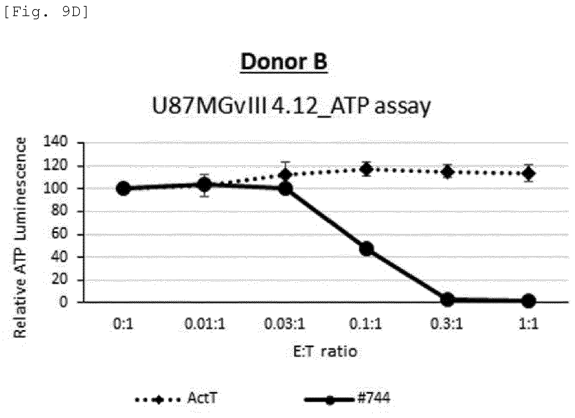

As a control, glioblastoma cell line U87MG cells not expressing EGFRviii were used in place of U87MGviii cells, and activated T cells were used in place of PRIME EGFRviii CAR-T cells for measurement in a similar manner. The results are illustrated in Figs. 9A to 9D.

The PRIME EGFRviii CAR-T cells exhibited strong cytotoxic activity in an EGFRviii expression specific manner.

### (Example 4) Evaluation of in vivo antitumor activity of anti-EGFRviiiCAR-T cells

### <Confirmation of expression of EGFRviii in glioblastoma cells U87MGviii 4.12>

### [Flow cytometry analysis]

Expression of EGFRviii in glioblastoma cells U87MGviii 4.12 (hereinafter, also simply referred to as "tumor cells") was confirmed for use in an assay to study cytotoxic activity and in preparing an animal model to study antitumor activity in vivo.

Specifically, a hybridoma culture supernatant of an anti-human EGFRviii antibody (clone 1B10-1A12) was added at 50 uL/sample, then an anti-mouse IgG antibody (Cat. No. 405308, manufactured by BioLegend, Inc.) labeled with allophycocyanin (APC) as a secondary antibody was stained at 50 ng/sample, and flow cytometry analysis was performed.

CytoFLEX S (manufactured by Beckman Coulter, Inc.) was used as the flow cytometer, and FlowJo software (manufactured by Tree Star, Inc.) was used for data analysis.

### (Results)

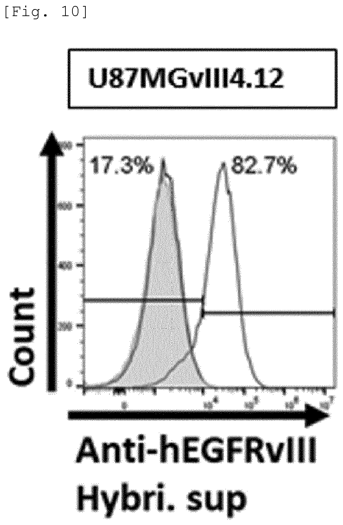

The results are illustrated in Fig. 10. 80% or more of tumor cells were confirmed to express EGFRviii.

### <Therapeutic effect using mouse tumor model>

A double-deficient mouse (NOG-lab/b2m dKO mouse) in which an NGO gene and a B2M gene were deficient was inoculated with 1.0 × 10⁶ glioblastoma cells U87MGviii 4.12 subcutaneously to prepare a tumor model mouse.

Ten days after subcutaneous inoculation, to five tumor model mice in each group, a Hank's balanced salt solution (HBSS) was administered intravenously as an untreated group, 1.25 × 10⁷ activated T cells were administered intravenously as an activated T cell administration group, and 1 × 10⁷ EGFRviii CAR-T cells (1.25 × 10⁷ cells from a cell group having a CAR expression ratio of 80%) were administered intravenously as a PRIME EGFRviii CAR-T cell (#744) administration group.

Thereafter, a tumor volume and a body weight of each of the model mice were measured twice a week, and a model mouse which decreased the body weight by 200 or a model mouse which was significantly weakened was dissected to confirm an anatomical photograph and presence or absence of tumor metastasis.

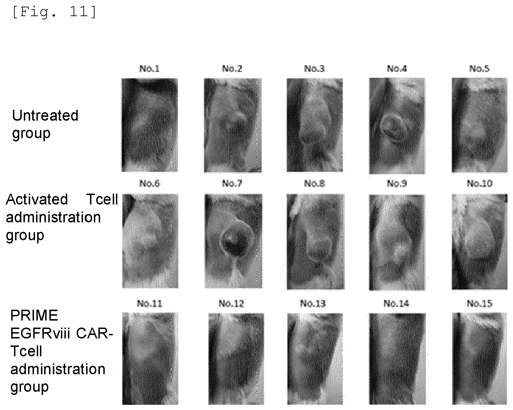

Photographs of backs of the tumor model mice inoculated with tumor cells are illustrated in Fig. 11.

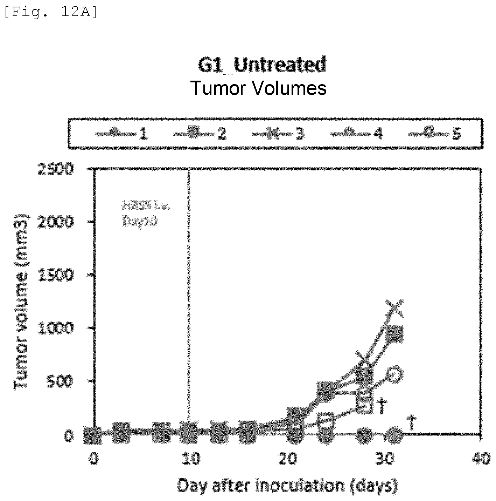

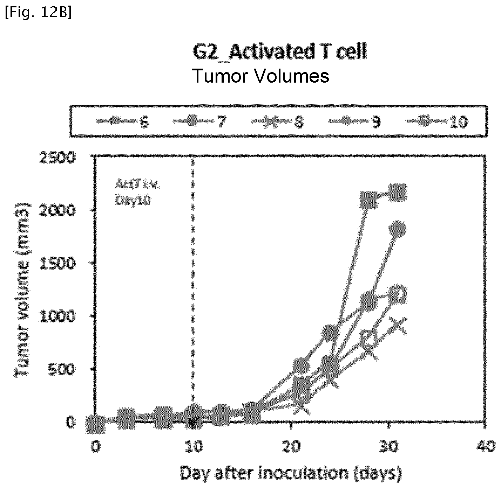

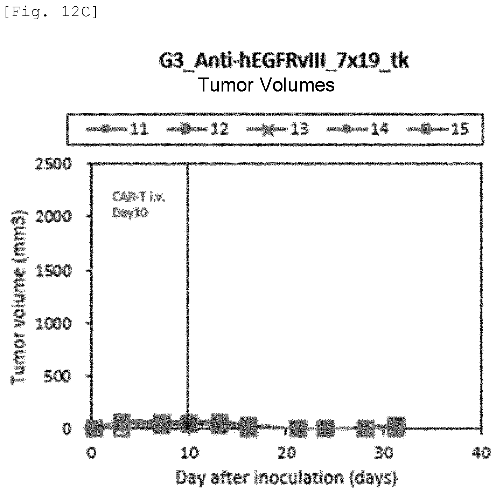

Results of changes in tumor volumes of the tumor model mice are illustrated in Figs. 12A to 12C. The horizontal axis represents the number of days after inoculation of tumor cells into mice (the day of inoculation into mice was day 0), and the vertical axis represents a tumor volume (major axis of tumor × (minor axis of tumor)²/2 (mm³)). The number of each piece of data is an individual identification number.

In the untreated group, the tumor volume increased except for one case in which tumor cells were not engrafted. In the activated T cell administration group, a significant increase in tumor volume was observed in each case. Meanwhile, in the PRIME EGFRviii CAR-T cell administration group, the tumor volume hardly increased.

As illustrated in Figs. 11 and 12A to 12C, it was confirmed that PRIME EGFRviii CAR-T cells (anti-EGFRviiiCAR-IL-7/CCL19-expressing T cells) exhibited excellent antitumor activity in tumor model mice.

## Claims

1. An anti-EGFRviii antibody which is an antibody or an antigen-binding fragment thereof selected from the group consisting of the following (a-1) to (a-3):
(a-1) an antibody or an antigen-binding fragment thereof including:
a heavy chain variable region including heavy chain variable regions CDR1, CDR2, and CDR3 each consisting of the amino acid sequence set forth in SEQ ID NO: 1 or 4; and
a light chain variable region including light chain variable regions CDR1, CDR2, and CDR3 each consisting ofthe amino acid sequence set forth in SEQ ID NO: 2, 3, 5, or 6, and
having binding ability to EGFRviii;
(a-2) an antibody or an antigen-binding fragment thereof including:
a heavy chain variable region consisting of an amino acid sequence having one or a plurality of amino acids mutated in the amino acid sequence set forth in SEQ ID NO: 1 or 4; and
a light chain variable region consisting of an amino acid sequence having one or a plurality of amino acids mutated in the amino acid sequence set forth in SEQ ID NO: 2, 3, 5, or 6, and
having binding ability to EGFRviii; and
(a-3) an antibody or an antigen-binding fragment thereof including:
a heavy chain variable region consisting of an amino acid sequence having 90% or more of sequence identity to the amino acid sequence set forth in SEQ ID NO: 1 or 4; and
a light chain variable region consisting of an amino acid sequence having 90% or more of sequence identity to the amino acid sequence set forth in SEQ ID NO: 2, 3, 5, or 6;
and
having binding ability to EGFRviii.

2. The anti-EGFRviii antibody according to claim 1, wherein the heavy chain variable region has T at position 21, F at position 24, F at position 54, K at position 59, S at position 67, K at position 73, T or K at position 77, and K or T at position 83 in the amino acid sequence set forth in SEQ ID NO: 1 or 4.

3. The anti-EGFRviii antibody according to claim 1 or 2, wherein the light chain variable region has M at position 4, E or G at position 16, Q at position 27, K at position 35, S at position 55, N at position 58, R at position 74, K at position 79, S at position 81, D at position 87, and V at position 99 in the amino acid sequence set forth in SEQ ID NO: 2, 3, 5, or 6.

4. The anti-EGFRviii antibody according to any one of claims 1 to 3, wherein
the heavy chain variable region includes the amino acid sequence set forth in SEQ ID NO: 1 and the light chain variable region includes the amino acid sequence set forth in SEQ ID NO: 2,
the heavy chain variable region includes the amino acid sequence set forth in SEQ ID NO: 1 and the light chain variable region includes the amino acid sequence set forth in SEQ ID NO: 3,
or
the heavy chain variable region includes the amino acid sequence set forth in SEQ ID NO: 4 and the light chain variable region includes the amino acid sequence set forth in SEQ ID NO: 5 or 6.

5. The anti-EGFRviii antibody according to any one of claims 1 to 4, wherein the antibody is a single chain variable fragment.

6. The anti-EGFRviii antibody according to claim 5, wherein the single chain variable fragment includes the light chain variable region, a peptide linker linking the heavy chain variable region and the light chain variable region to each other, and the heavy chain variable region sequentially from an N-terminus.

7. The anti-EGFRviii antibody according to claim 5, wherein the single chain variable fragment is a polypeptide selected from the group consisting of the following (I) to (III) :
(I) a polypeptide including an amino acid sequence selected from the group consisting of SEQ ID NOs: 12 to 18 and 38;
(II) a polypeptide including an amino acid sequence having 70% or more of sequence identity to the amino acid sequence selected from the group consisting of SEQ ID NOs: 12 to 18 and 38, and having binding ability to EGFRviii; and
(III) a polypeptide including an amino acid sequence having one or a plurality of amino acids mutated in the amino acid sequence selected from the group consisting of SEQ ID NOs: 12 to 18 and 38, and having binding ability to EGFRviii.

8. A polypeptide comprising a target antigen-binding region, a transmembrane region, and a signal transduction region,
wherein the target antigen-binding region includes the anti-EGFRviii antibody according to any one of claims 1 to 7.

9. The polypeptide according to claim 8, which is a chimeric antigen receptor.

10. A cell expressing the polypeptide according to claim 8 or 9.

11. The cell according to claim 10, further expressing at least one of IL-7 and CCL19.

12. The cell according to claim 10 or 11, wherein the cell is an immune cell.

13. The cell according to claim 12, wherein the immune cell is a T cell.

14. A polynucleotide comprising a nucleotide sequence encoding the polypeptide according to claim 8 or 9.

15. The polynucleotide according to claim 14, further comprising at least one of a nucleotide sequence encoding IL-7 and a nucleotide sequence encoding CCL19.

16. A vector comprising the polynucleotide of claim 14 or 15.

17. The vector according to claim 16, further comprising at least one of a nucleotide sequence encoding IL-7 and a nucleotide sequence encoding CCL19.

18. A method for producing a cell expressing a polypeptide, the method comprising introducing the polynucleotide according to claim 14 or 15 into a cell.

19. A method for producing a cell expressing a polypeptide, the method comprising introducing the vector according to claim 16 or 17 into a cell.

20. A pharmaceutical composition comprising the cell according to any one of claims 10 to 13.

21. The pharmaceutical composition according to claim 20, which is a pharmaceutical composition for treating or preventing a tumor.
